(19) Europäisches Patentamt
European Patent Office
Office européen des brevets

(11) Publication number: **0 497 988 A1**

(12) # EUROPEAN PATENT APPLICATION published in accordance with Art. 158(3) EPC

(21) Application number: **91914756.1**

(51) Int. Cl.[5]: **C07H 15/04**, A61K 31/70

(22) Date of filing: **23.08.91**

(86) International application number:
**PCT/JP91/01122**

(87) International publication number:
**WO 92/03453 (05.03.92 92/06)**

(30) Priority: **23.08.90 JP 222187/90**
**31.08.90 JP 228306/90**
**31.08.90 JP 228307/90**
**07.09.90 JP 235649/90**
**30.11.90 JP 335713/90**
**30.04.91 JP 99050/91**
**23.08.91 JP 211833/91**

(43) Date of publication of application:
**12.08.92 Bulletin 92/33**

(84) Designated Contracting States:
**CH DE FR GB IT LI**

(71) Applicant: **DAINIPPON INK AND CHEMICALS, INC.**
**35-58, Sakashita 3-chome**
**Itabashi-ku, Tokyo 174(JP)**

(72) Inventor: **SHOJI, Tadao, 6-36-13, Ojidai**
**Sakura-shi**
**Chiba 285(JP)**
Inventor: **TAKAHASHI, Nahoko, 4-3-2-203, Takahama**
**Chiba-shi**
**Cciba 260(JP)**
Inventor: **IKUSHIMA, Naoya, 1550-2-2-203, Mutsusaki**
**Sakura-shi**
**Chiba 285(JP)**
Inventor: **KATSURAYA, Kaname, 1550-2-1-204, Mutsusaki**
**Sakura-shi**
**Chiba 285(JP)**
Inventor: **URYU, Toshiyuki, 3-5-26-519, Aoi**
**Adachi-ku**
**Tokyo 120(JP)**
Inventor: **YOSHIDA, Takashi, 2-16-13, Nishimagome**
**Ohta-ku**
**Tokyo 143(JP)**
Inventor: **NISHIHASHI, Hideji, 2-23-9, Oosakidai**
**Sakura-shi**
**Chiba 285(JP)**
Inventor: **YAMAMOTO, Naoki, 501 Haramachijutaku**
**3-11-17, Ebisuminami Shibuya-ku**
**Tokyo 150(JP)**
Inventor: **NAKASHIMA, Hideki, 16-405, Musashinojutaku**
**4-3, Gakuen Musashimurayama-shi**
**Tokyo 180(JP)**
Inventor: **SHIGETA, Shiro, 147-28, Aza-kubouchi Ohmori**
**Fukushima-shi**
**Fukushima 960-11(JP)**

(74) Representative: **Hansen, Bernd, Dr. Dipl.-Chem. et al**
**Hoffmann, Eitle & Partner Patent- und Rechtsanwälte Arabellastrasse 4 Postfach 81 04 20**
**W-8000 München 81(DE)**

(54) **ANTIVIRAL AGENT.**



Rank Xerox (UK) Business Services

(57) A sulfated alkyl oligosaccharide prepared by substituting the hydroxyl group at the 1-position of the terminal sugar of an oligosaccharide with an alkyl group and sulfating at least 14.3 % of the remaining hydroxyl groups of the sugar moiety; a physiologically acceptable salt thereof; and an antiviral agent containing the same as the active ingredient. This agent has a significantly reduced toxicity and an antiviral activity against a wide variety of DNA and RNA viruses irrespectively of whether the virus has an envelope or not and a particularly excellent antiviral activity against AIDS virus.

FIG.1

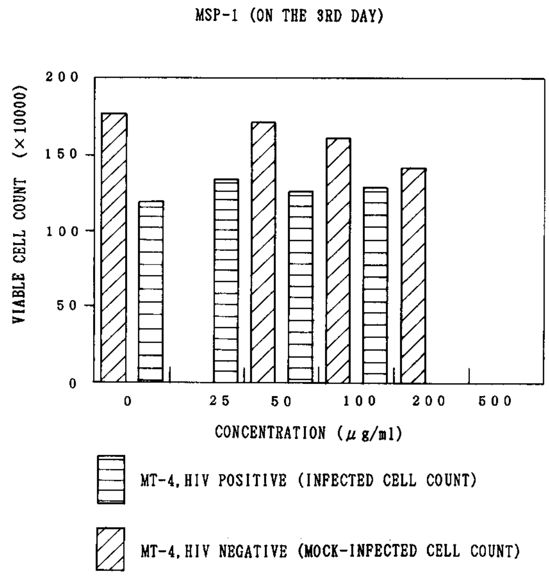

[Field of the Invention]

The present invention relates to alkyl oligosaccharide sulfate and to antiviral agents with alkyl oligosaccharide sulfate as the active component thereof having a broad antiviral spectrum, especially those with activity against the AIDS virus (HIV, the Human Immunodeficiency Virus).

[Background Art]

Concerning saccharide sulfate compounds, monosaccharide sulfates and polysaccharide sulfates have long been known and many references relating to these compounds are available. For example, saccharide sulfates are described in Advances in Carbohydrate Chemistry, volume 20, p. 183; and Volume 25, p. 407.

As for alkyl saccharides, the reaction products obtained by alkylating saccharides have been described as being of use as surface activating agents in the 1934 United States Patent, No. 1,951,784; the 1989 United States Patent No. 4,806,275, and European Patent Application, Publication No. 280715. However, in these documents, the described reaction products have only a utility as low cost surface activating agents, and for this reason, isolation of the constituent compounds was not carried out. Thus, each of these references describe mixtures of compounds and in none are the chemical structures of the constituents of the mixtures clearly elucidated.

As for compounds having antiviral activity, numerous studies exist reporting the utility of polysaccharide sulfate compounds as therapeutic agents for AIDS (Acquired Immunodeficiency Syndrome), for example, Japanese Patent Application, First Publication Serial No. Showa 62-215529; Hideki Nakamura, et al., Jpn. J. Cancer Res. (Gann), 78, 1164 (1987); Osamu Yoshida, et al., Biochemical Pharmacology, 37 2887-2891 (1988); and Japanese patent Application, First Publication Serial No. Heisei 1-103601. However, a number of problems associated with the administration of these agents have recently come to light, such as poor systemic absorption due to the molecular weight of polysaccharide sulfates which range from tens of thousands to far greater, difficulties with oral administration, antigenic properties and anticoagulation effects. Additionally, because the widely used nucleic acid antiviral agent AZT (dideoxyazidothymine) also has considerable side effects, there has been a demand for the development of a new pharmacological agent having low toxicity.

The object of the present invention is to provide a new alkyl oligosaccharide sulfate and an antiviral agent with alkyl oligosaccharide sulfate as the active component thereof having easy systemic absorption, low toxicity and a broad antiviral spectrum, especially against the AIDS virus (HIV).

[Summary of the Invention]

The alkyl oligosaccharide sulfate of the present invention consists of an oligosaccharide formed from a single monosaccharide or a combination of different types of monosaccharides, bonded by glycoside bonds, in which a hydroxyl group at the position-1 carbon atom has been replaced with a straight or branched alkyl group, and wherein at least 14.3% of the remaining hydroxyl groups have been replaced with sulfate groups.

The antiviral agent of the present patent application also includes the above described oligosaccharide sulfate of the present invention, physiologically acceptable salts thereof and preparations including the alkyl oligosaccharide sulfate of the present invention and/or physiologically acceptable salts thereof as effective components.

The antiviral agent of the present invention is more easily systemically absorbed and comparatively more resistant to in vivo degradation than those consisting of polysaccharides sulfates. Furthermore, the antiviral agent of the present invention has an exceedingly wide antiviral spectrum with activity against both DNA viruses and RNA viruses and viruses with or without envelopes, and has excellent antiviral activity against the AIDS virus.

[Brief Description of the Drawings]

Fig. 1 is a graph illustrating the number of viable cells from among HIV-infected and non-infected (mock-infected) cells to which MPS-1 had been tested on the third day as an example of an antiviral agent in accordance with the present invention.

Fig. 2 is a graph illustrating the number of viable cells from among HIV-infected and non-infected (mock infected) cells to which MPS-1 had been tested on the sixth day as an example of an antiviral agent in accordance with the present invention.

Fig. 3 is a graph illustrating the number of viable cells from among HIV-infected and non-infected (mock infected) cells to which MPS-2 had been tested on the third day as an example of an antiviral agent in accordance with the present invention.

Fig. 4 is a graph illustrating the number of viable cells from among HIV-infected and non-infected (mock infected) cells to which MPS-2 had been tested on the sixth day as an example of an antiviral agent in accordance with the present invention.

Fig. 5 is a graph illustrating the number of viable cells from among HIV-infected and non-infected (mock infected) cells to which MPS-3 had been tested on the third day as an example of an antiviral agent in accordance with the present invention.

Fig. 6 is a graph illustrating the number of viable cells from among HIV-infected and non-infected (mock infected) cells to which MPS-3 had been tested on the sixth day as an example of an antiviral agent in accordance with the present invention.

Fig. 7 is a graph illustrating the number of viable cells from among HIV-infected and non-infected (mock infected) cells to which LPS-1 had been tested as an example of an antiviral agent of the present invention.

Fig. 8 is a graph illustrating the number of viable cells from among HIV-infected and non-infected (mock infected) cells to which LPS-1 had been tested as an example of an antiviral agent of the present invention.

[Detailed Description of the Preferred Embodiments]

For the constituent monosaccharides which make up the component of the alkyl oligosaccharide for alkyl oligosaccharide sulfates in accordance with the present invention, a single monosaccharide or a combination of different monosaccharides can be used, selected from the group including glucose, galactose, mannose, talose, idose, altrose, allose, gulose, xylose, arabinose, rhamnose, fucose, and fructose.

From the standpoint of antiviral activity, the number of individual monosaccharide units optimally used to form the alkyl oligosaccharide component of alkyl oligosaccharide sulfates in accordance with the present invention is variable, depending on the type or types of monosaccharides used therefor. Ordinarily, however, a number of 30 individual monosaccharides or less is desirable when consideration is given to physiological compatibility and to the anticoagulant effect seen in the case of polysaccharide sulfates. for this reason, for the oligosaccharide component of the alkyl oligosaccharide sulfates of the present invention, those having from 2 to 30 constituent monosaccharides are used.

When alkyl oligosaccharide sulfates in accordance with the present invention, based on the consideration of antiviral activity, types of glycoside bonds between the constituent monosaccharides including (1 → 2) bonds, (1 → 3) bonds, (1 → 4) bonds, and (1 → 6) bonds, and all available bonds, although those with α(1 → 4) type bonds readily undergo in vivo degradation, for which reason the antiviral activity thereof suffers. When those having α(1 → 4) type glucoside bonds is administered as an antiviral therapeutic agent, it must be administered at a higher total daily dose compared with a lower rate of metabolism. Based on these consideration, alkyl oligosaccharide sulfates having β-glycoside bonds are generally preferred over those having α-glycoside bonds which tend to be comparatively more susceptible to in vivo degradation. Furthermore, alkyl oligosaccharide sulfate in accordance with the present invention having (1 → 3) or (1 → 6) glycoside bonds are generally preferred over those with (1 → 4) bonds. In greater detail, preferred oligosaccharides include β-(1 → 3) oligoglucose (oligosaccharide consisting of glucose units sequentially bonded via β conformation of ether linkages between the hydroxy group of a position-1 carbon atom on one glucose and a position-3 carbon atom on the next), such as those derived from a breakdown of polysaccharides such as curdlan and laminaran; and those such as the oligosaccharide formed of galactose successively bonded via β(1 → 4) bonds to the position-4 carbon atom of the galactose portion thereof, an additional galactose is bonded thereto via a β (1 → 4) bond. Also usable are oligosaccharides such as oligomaltose with α(1 → 4) bonds; as well as mannose type oligosaccharides; α(1 → 6), β(1 → 6) oligoglucose; and the various oligosaccharides such as xylan types, schizophyllan types, lentinan types, and galactan types.

Concerning the alkyl portion of alkyl oligosaccharide sulfates in accordance with the present invention, the position whereat the alkyl group is bonded is particularly important, such that the alkyl group must be bonded to the oxygen atom at the position-1 of a terminal reducing sugar. The present inventors have carried out various investigations concerning the reaction conditions under which the alkyl group is introduced into the oligosaccharide. For example, using an alkyl halide under basic conditions to introduce an alkyl functionality to an oligosaccharide group, the alkyl group was mainly introduced to primary hydroxyl groups, but none was introduced to the position-1 hydroxy group of the terminal reducing sugar. In the following step in which sulfation was carried out, side reactions took place which were believed to be a consequence of the fact that the hydroxy group of the reducing end of the terminal sugar remained free. Because many decomposition reactions occurred, it was not possible to obtain highly pure alkyl oligosaccharide sulfates. Additionally, by comparison of the antiviral activity of the compounds prepared in comparative example 3 and experimental example 8 which are described later, it is possible to verify the importance of alkylation of hydroxyl groups at the position-1 carbon atom of terminal reducing sugars.

Although the alkyl group in the alkyl oligosaccharide sulfates in accordance with the present invention can be branched or straight-chain, generally when long alkyl chain groups are used, reaction yield decreases, and for this reason the alkyl group should usually have 22 or fewer carbon atoms, and more preferably, should have 8 to 18 carbon atoms.

Concerning methods whereby an alkyl group can be introduced at the position-1 oxygen atom of a terminal reducing sugar, ordinarily glycosidation reaction can simply be carried out. To do so, methods which can be used include those involving reaction of the peracetate of a saccharide with an alcohol under acidic condition, methods wherein after the saccharide peracetate is brominated via a Koenigs-Knorr reaction and then allowed to react with an alcohol using silver chloride or mercury chloride, methods involving the reaction of a glycosyl bromide with an alcohol in the presence of tetraethyl ammonium bromide, methods in which a saccharide imidate is reacted with an alcohol, as well as various other such methods. Among these methods, generally those involving reactions with an alcohol under acidic conditions can be most easily carried out. Thus, with an alkyl glycosaccharide peracetate which can be obtained, by an ordinary method using sodium methoxide, ammonia or the like to carry out deacetylation, an alkyl oligosaccharide (alkyl glycoside) can be obtained.

Alcohols which can be used in the alkylation reaction include straight or branched alkyl alcohol, although from the viewpoint of reactivity, primary alcohols are preferable. For the alkylation reaction used to prepare alkyl oligosaccharide sulfates in accordance with the present invention, together with an acidic catalyst, an alcohol which can be used include methanol, ethanol, n-propanol, 2-methylpropanol, 2,2-dimethyl-1-propanol, n-butanol, n-pentanol, 2-methyl-1-butanol, 3-methyl-1-butanol, n-hexanol, 2-methyl-1-pentanol, 3-methyl-1-pentanol, 4-methyl-1-pentanol, 3,3-dimethyl-1-butanol, n-heptanol, 2-methyl-1-pentanol, n-octanol, 2-ethyl-1-hexanol, 3,7-dimethyl-1-octonol, n-nananol, 6-methyl-1-octanol, n-decanol, n-unidecanol, n-dodecanol, n-tridecanol, n-tetradecanol, n-pentadecanol, n-hexadecanol, n-heptadecanol, n-octadecanol, n-nonadecanol, n-eicosanol, n-docosanol, and others.

For the sulfate prepared from an alkyl oligosaccharide using a sulfating agent, it is possible to produce a physiologically acceptable sodium salt, potassium salt, magnesium salt or some other type of physiologically acceptable salt as the final product.

For the sulfation, sulfating agents such as sulfur trioxide pyridine complex, piperidine sulfate (1-piperidine sulfonic acid), and chlorosulfonic acid can be used. By allowing the hydroxyl groups of an alkyl oligosaccharide to react with such a sulfating agent in an aprotic solvent, then treating with an aqueous solution of a metal hydroxide, the desired compound can be obtained.

To express the extent of sulfate conversion of an oligosaccharide sulfate, the term "sulfation index" is sometimes used. The sulfation index represents the number of hydroxyl groups which have actually been converted to a sulfate group in the sulfation divided by the number of constituent monosaccharides in the oligosaccharide. Thus, the sulfation index expresses the ratio of the number of sulfated hydroxyl groups to the number of monosaccharide units. An example of the calculation of the sulfation index is given below.

For example, in the case of an alkyl oligosaccharide consisting of 5 six-carbon monosaccharides, the maximum sulfation index of 3.2 is obtained when each hydroxyl group for which sulfation conversion is possible has been converted to a sulfate group, as is shown in equation 1 below.

$$(3 + 3 + 3 + 3 + 4) / 5 = 3.2 \qquad \text{Equ.1}$$

For the alkyl oligosaccharide in this case, if only two of the hydroxyl groups have been converted to sulfate groups, the calculated sulfation index is 0.4, as is shown in equation 2 below.

$$2 / 5 = 0.4 \qquad \text{Equ.2}$$

Another index for the extent of sulfate conversion is the sulfation ratio. The sulfation ratio, which is the ratio of the number of sulfated hydroxyl groups to the total number of reactable hydroxyl groups expressed as a percentage value, is also frequently used.

For the case described in connection with equation 1 above, where all of the reactable hydroxyl groups of an alkyl oligosaccharide consisting of 5 six-carbon monosaccharides have been converted to sulfate groups, the calculated sulfation ratio is 100%, as is shown in equation 3 below.

(16 / 16) X 100% = 100% Equ.3

for the case described in connection with equation 2 above, where only two of the reactable hydroxyl groups of the alkyl oligosaccharide have been converted to sulfate groups, the calculated sulfation ratio is 12.5%, as shown in equation 4 below.

(2 / 16) X 100% = 12.5% Equ.4

In the case of an alkyl oligosaccharide consisting of 2 six-carbon monosaccharides having seven reactable hydroxyl groups, when only one of the hydroxyl groups for which sulfate conversion is possible has been converted to a sulfate group, the calculated sulfation ratio is 14.3% as shown in equation 5 below, and the sulfation index is 0.5 as shown in equation 6 below.

(1 / 7) X 100% = 14.3% Equ.5

1 / 2 = 0.5 Equ.6

The extent of sulfation of an alkyl oligosaccharide is expressed as thus described. In order to achieve the desired sulfation index (sulfation ratio), the amount of alkyl oligosaccharide is determined in consideration of the equivalent ratio of alkyl oligosaccharide hydroxyl groups to the sulfating agent. When an alkyl oligosaccharide sulfate is to be used as an antiviral agent, a sulfation ratio of at least 14.3%, that is, a sulfation index of at least 0.5 and as large as possible, is desirable.

Below, examples of alkyl oligosaccharide sulfates in accordance with the present invention will be presented. The use of the term "sulfate" in the following list of alkyl oligosaccharide sulfates indicates a sulfation ratio of 14.3% or greater, that is to say, a sulfation index of no less than 0.5.

hexyl β-D-glucopyranosyl (1 → 3)-β-D-glucopyranoside sulfate
heptyl β-D-glucopyranosyl (1 → 3)-β-D-glucopyranoside sulfate
octyl β-D-glucopyranosyl (1 → 3)-β-D-glucopyranoside sulfate
nonyl β-D-glucopyranosyl (1 → 3)-β-D-glucopyranoside sulfate
decyl β-D-glucopyranosyl (1 → 3)-β-D-glucopyranoside sulfate
dodecyl β-D-glucopyranosyl (1 → 3)-β-D-glucopyranoside sulfate
hexadecyl β-D-glucopyranosyl (1 → 3)-β-D-glucopyranoside sulfate
octadecyl β-D-glucopyranosyl (1 → 3)-β-D-glucopyranoside sulfate
eicosanyl β-D-glucopyranosyl (1 → 3)-β-D-glucopyranoside sulfate
docosanyl β-D-glucopyranosyl (1 → 3)-β-D-glucopyranoside sulfate
2-ethylhexyl β-D-glucopyranosyl (1 → 3)-β-D-glucopyranoside sulfate
3-methylpentyl β-D-glucopyranosyl (1 → 3)-β-D-glucopyranoside sulfate
hexyl β-D-glucopyranosyl (1 → 3)-(β-D-glucopyranol 1 → 3))$_n$-β-D-glucopyranoside sulfate
heptyl β-D-glucopyranosyl (1 → 3)-(β-D-glucopyranol 1 →3))$_n$-β-D-glucopyranoside sulfate
octyl β-D-glucopyranosyl (1 → 3)-(β-D-glucopyranol 1 → 3))$_n$-β-D-glucopyranoside sulfate
nonyl β-D-glucopyranosyl (1 → 3)-(β-D-glucopyranol 1 → 3))$_n$-β-D-glucopyranoside sulfate
decyl β-D-glucopyranosyl (1 → 3)-(β-D-glucopyranol 1 → 3))$_n$-β-D-glucopyranoside sulfate
dodecyl β-D-glucopyranosyl (1 → 3)-(β-D-glucopyranol 1 → 3))$_n$-β-D-glucopyranoside sulfate
hexadecyl β-D-glucopyranosyl (1 → 3)-(β-D-glucopyranol 1 → 3))$_n$-β-D-glucopyranoside sulfate
octadecyl β-D-glucopyranosyl (1 → 3)-(β-D-glucopyranol 1 → 3))$_n$-β-D-glucopyranoside sulfate
eicosanyl β-D-glucopyranosyl (1 → 3)-(β-D-glucopyranol 1 → 3))$_n$-β-D-glucopyranoside sulfate
docosanyl β-D-glucopyranosyl (1 → 3)-(β-D-glucopyranol 1 → 3))$_n$-β-D-glucopyranoside sulfate
2-ethylhexyl β-D-glucopyranosyl (1 → 3)-(β-D-glucopyranol 1 → 3))$_n$-β-D-glucopyranoside sulfate
3-methylpentyl β-D-glucopyranosyl (1 → 3)-(β-D-glucopyranol 1 → 3))$_n$-β-D-glucopyranoside sulfate
In these examples, n indicates and interger from 1 to 28.
hexyl β-D-galactopyranosyl (1 → 4)-(β-D-galactopyranosyl 1 → 4))-β-D-glucopyranoside sulfate
heptyl β-D-galactopyranosyl (1 → 4)-(β-D-galactopyranosyl 1 → 4))-β-D-glucopyranoside sulfate
octyl β-D-galactopyranosyl (1 → 4)-(β-D-galactopyranosyl 1 → 4))-β-D-glucopyranoside sulfate
nonyl β-D-galactopyranosyl (1 → 4)-(β-D-galactopyranosyl 1 → 4))-β-D-glucopyranoside sulfate
decyl β-D-galactopyranosyl (1 → 4)-(β-D-galactopyranosyl 1 → 4))-β-D-glucopyranoside sulfate
dodecyl β-D-galactopyranosyl (1 → 4)-(β-D-galactopyranosyl 1 → 4))-β-D-glucopyranoside sulfate
hexadecyl β-D-galactopyranosyl (1 → 4)-(β-D-galactopyranosyl 1 → 4))-β-D-glucopyranoside sulfate

octadecyl β-D-galactopyranosyl (1 → 4)-(β-D-galactopyranosyl 1 → 4))-β-D-glucopyranoside sulfate
eicosanyl β-D-galactopyranosyl (1 → 4)-(β-D-galactopyranosyl 1 → 4))-β-D-glucopyranoside sulfate
docosanyl β-D-galactopyranosyl (1 → 4)-(β-D-galactopyranosyl 1 → 4))-β-D-glucopyranoside sulfate
2-ethylhexyl β-D-galactopyranosyl (1 → 4)-(β-D-galactopyranosyl 1 → 4))-β-D-glucopyranoside sulfate
3-methylpental β-D-galactopyranosyl (1 → 4)-(β-D-galactopyranosyl 1 → 4))-β-D-glucopyranoside sulfate
hexyl β-D-galactopyranosyl (1 → 4)-(β-D-galactopyranosyl 1 → 4)) $_n$-β-D-glucopyranoside sulfate
heptyl β-D-galactopyranosyl (1 → 4)-(β-D-galactopyranosyl 1 → 4))$_n$-β-D-glucopyranoside sulfate
octyl β-D-galactopyranosyl (1 → 4)-(β-D-galactopyranosyl 1 → 4))$_n$-β-D-glucopyranoside sulfate
nonyl β-D-galactopyranosyl (1 → 4)-(β-D-galactopyranosyl 1 → 4))$_n$-β-D-glucopyranoside sulfate
decyl β-D-galactopyranosyl (1 → 4)-(β-D-galactopyranosyl 1 → 4))$_n$-β-D-glucopyranoside sulfate
dodecyl β-D-galactopyranosyl (1 → 4)-(β-D-galactopyranosyl 1 → 4))$_n$-β-D-glucopyranoside sulfate
hexadecyl β-D-galactopyranosyl (1 → 4)-(β-D-galactopyranosyl 1 → 4))$_n$-β-D-glucopyranoside sulfate
octadecyl β-D-galactopyranosyl (1 → 4)-(β-D-galactopyranosyl 1 → 4))$_n$-β-D-glucopyranoside sulfate
eicosanyl β-D-galactopyranosyl (1 → 4)-(β-D-galactopyranosyl 1 → 4))$_n$-β-D-glucopyranoside sulfate
docosanyl β-D-galactopyranosyl (1 → 4)-(β-D-galactopyranosyl 1 → 4))$_n$-β-D-glucopyranoside sulfate
2-ethylhexyl β-D-galactopyranosyl (1 → 4)-(β-D-galactopyranosyl 1 → 4))$_n$-β-D-glucopyranoside sulfate
3-methylpentyl β-D-galactopyranosyl (1 → 4)-(β-D-galactopyranosyl 1 → 4))$_n$-β-D-glucopyranoside sulfate
In these examples, n indicates an interger from 1 to 28.
hexyl α-D-glucopyranosyl (1 → 4)-(β-D-glucopyranoside sulfate
heptyl α-D-glucopyranosyl (1 → 4)-(β-D-glucopyranoside sulfate
octyl α-D-glucopyranosyl (1 → 4)-(β-D-glucopyranoside sulfate
nonyl α-D-glucopyranosyl (1 → 4)-(β-D-glucopyranoside sulfate
decyl α-D-glucopyranosyl (1 → 4)-(β-D-glucopyranoside sulfate
dodecyl α-D-glucopyranosyl (1 → 4)-(β-D-glucopyranoside sulfate
hexadecyl α-D-glucopyranosyl (1 → 4)-(β-D-glucopyranoside sulfate
octadecyl α-D-glucopyranosyl (1 → 4)-(β-D-glucopyranoside sulfate
eicosanyl α-D-glucopyranosyl (1 → 4)-(β-D-glucopyranoside sulfate
docosanyl α-D-glucopyranosyl (1 → 4)-(β-D-glucopyranoside sulfate
2-ethylhexyl α-D-glucopyranosyl (1 → 4)-(β-D-glucopyranoside sulfate
3-methylpentyl α-D-glucopyranosyl (1 → 4)-(β-D-glucopyranoside sulfate
hexyl α-D-glucopyranosyl (1 → 4)-(α-D-glucopyranol (1 → 4))$_n$-β-D-glucopyranoside sulfate
heptyl α-D-glucopyranosyl (1 → 4)-(α-D-glucopyranol (1 → 4))$_n$-β-D-glucopyranoside sulfate
octyl α-D-glucopyranosyl (1 → 4)-(α-D-glucopyranol (1 → 4))$_n$-β-D-glucopyranoside sulfate
nonyl α-D-glucopyranosyl (1 → 4)-(α-D-glucopyranol (1 → 4))$_n$-β-D-glucopyranoside sulfate
decyl α-D-glucopyranosyl (1 → 4)-(α-D-glucopyranol (1 → 4))$_n$-β-D-glucopyranoside sulfate
dodecyl α-D-glucopyranosyl (1 → 4)-(α-D-glucopyranol (1 → 4))$_n$-β-D-glucopyranoside sulfate
octadecyl α-D-glucopyranosyl (1 → 4)-(α-D-glucopyranol (1 → 4))$_n$-β-D-glucopyranoside sulfate
eicosanyl α-D-glucopyranosyl (1 → 4)-(α-D-glucopyranol (1 → 4))$_n$-β-D-glucopyranoside sulfate
docosanyl α-D-glucopyranosyl (1 → 4)-(α-D-glucopyranol (1 → 4))$_n$-β-D-glucopyranoside sulfate
2-ethylhexyl α-D-glucopyranosyl (1 → 4)-(α-D-glucopyranol (1 → 4))$_n$-β-D-glucopyranoside sulfate
3-methylpentyl α-D-glucopyranosyl (1 → 4)-(α-D-glucopyranol (1 → 4))$_n$-β-D-glucopyranoside sulfate
In these examples, n indicates an integer from 1 to 28.

The method of production of an alkyl oligosaccharide sulfate of the present invention will now be described in detail. Although in the following description, an alkyl oligosaccharide intermediate is prepared through four step processes, the method of production thereof is in no way so limited.

[Step 1] Acetylation of oligosaccharide

According to ordinary methods, the hydroxyl groups of an oligosaccharide are acetylated. After an oligosaccharide is added to pyridine, acetic anhydride is added, and stirring at room temperature is carried out for 10 to 20 hours allowing the reaction to go to completion. Alternatively, an oligosaccharide can be acetylated using sodium acetate and acetic anhydride. Additionally, acetylation methods employing sodium acetate can be used.

[Step 2] Alkylation (glycosidation)

Of various alkylation processes available now, the one involving reactions with alcohol in the presence of an acid as a catalyst is the most simple. For example, an oligosaccharide peracetate, as a product of acetylation of oligosaccharide in Step 1, is reacted with an alcohol in an adequate solvent, such as toluene, methylene chloride or 1,2-dichloroethylene, in the presence of a Lewis acid catalyst, such as ferric chloride, zinc chloride, stannic chloride or boron trifluoride ether, either alone or in combination. The ratio of a Lewis acid catalyst to an oligosaccharide peracetate is preferably in a range between 0.1 and 2.0 molar equivalents, more preferably between 0.1 and 1.0 molar equivalents. The ratio of an alcohol to an oligosaccharide peracetate is preferably in a range between 1.0 and 5.0 molar equivalents, more preferably between 1.0 and 3.0 molar equivalents. The reactions between an oligosaccharide peracetate and an alcohol in the presence of a Lewis acid catalyst are effected in an inert gas atmosphere at a temperature preferably in a range between room temperature and the boiling point of a solvent, more preferably from 20°C to 80°C. The optimum reaction time, although varying depending on the types of oligosaccharide and alcohol used, is generally in a range between 2 and 30 hours, more generally between 3 and 15 hours. After the reaction, the reaction mixture is poured into ice water and extracted with organic solvent not miscible with water, is washed and dehydrated to produce the organic layer crude product, which is purified by chromatography using silica gel, and if necessary reprecipitation or recrystallization process can be used to produce the final product. The process that uses a Lewis acid catalyst produces a $\beta$-anomer ($\beta$-glycoside) as the main product. It is possible to adjust the $\alpha$-anomer/$\beta$-anomer ratio by selecting a suitable process and catalyst or by manipulating the operating conditions.

[Step 3] Deacetylation

Step 3 is the deacetylation process in which an alkyl oligosaccharide peracetate prepared in Step 2 is dissolved in an adequate solvent, such as methanol, to which a sodium methoxide/methanol solution is added, and the mixture is stirred at room temperature, generally for 1 to 50 hours, and most commonly for 1 to 24 hours. It should be noted that precipitates may separate out from the reaction solution during the above process depending on the length of the alkyl group; such a phenomenon may be observed when the length of the saccharide chain of the oligosaccharide used in this process exceeds roughly 6 to 7 saccharides. These precipitates are mostly of alkyl oligosaccharides which have been deacetylated. There are sometimes residual compounds present in the system which are not completely deacetylated and contain one or more acetyl groups bonding to the saccharide chain. It is therefore preferable to completely deacetylate an alkyl oligosaccharide peracetate in the presence of a sufficient quantity of sodium methoxide in the sodium methoxide/methanol solution, in a range between 0.1 and 1.0 molar equivalents for the acetyl group. The reaction mixture is then treated with a hydrogen-ion-type cation-exchange resin which substitutes hydrogen ions for sodium ions in order to an produce alkyl oligosaccharide as a target product. This product can be further refined as necessary by recrystallization or reprecipitation. A methanol solution saturated with ammonia gas may be used in place of sodium methoxide.

[Step 4] Sulfation

There are many sulfation processes established so far, any of which is useful for the present invention. This specification describes the sulfur trioxide/pyridine complex process as one of the sulfation processes.

An alkyl oligosaccharide is dissolved in pyridine to which a sulfur trioxide pyridine complex is added in a necessary quantity, preferably 1.2 to 3 molar equivalents for the hydroxide group in the saccharide when complete sulfation is desired. The reactions take place in an inert gas atmosphere, preferably at a temperature in a range between room temperature and 100°C, more preferably from 50°C to 85°C, for 0.5 to 5 hours, more preferably for 0.5 to 2 hours. The precipitates which are formed are separated from pyridine, and after being washed with pyridine, the separated precipitates are dissolved in an adequate quantity of ion-exchanged water at room temperature, to which an aqueous solution of barium hydroxide is added in order to adjust the solution to a pH level of 7 to 8. The precipitates formed during this process are removed by centrifugal separation. The precipitate-free solution is then passed through a column packed with a sodium-ion-type ion-exchanging resin (Amberlite IR-120B (Trademark)), and the reaction mixture is evaporated to dryness. To the residue is added ion-exchanged water and an appropriate organic solvent, such as acetone. The precipitates formed are collected to produce an alkyl oligosaccharide sulfate as the target product. An alternate method uses sodium hydroxide in place of barium hydroxide to adjust the solution to a pH level of 9 to 10. After the solution is concentrated and solidified, at 40°C or less under a vacuum, the residue is dissolved in water to form an aqueous solution to separate surplus inorganic salts by means of ultrafiltration, a semipermeable membrane, a Maikuroashiraiza (an electric ion-exchanging membrane, supplied by Asahi Chemical Industries), or an ion-exchanging resin column. Methanol, acetone, or the like is added to the above aqueous solution to precipitate sodium salts of an alkyl oligosaccharide sulfate as the target product. Almost the same procedure may be used for piperdine sulfonic acid to produce the target product.

The antiviral agent of the present invention comprises alkyl oligosaccharide sulfates as the effective component formed from a single monosaccharide or a combination of different types of monosaccharides that are bonded by glycoside bonds, wherein the hydrogen atom in the hydroxyl group at the position-1 where the number one carbon atom at the terminal of the oligosaccharide molecule has been replaced with a straight or branched alkyl group, and wherein at least 14.3% of the remaining hydroxyl groups have been replaced with the sulfate group. The antiviral agent of the present invention also includes those comprising the physiologically acceptable salt as the effective component of the above-described alkyl oligosaccharide sulfate.

More particularly, the antiviral agent of the present invention comprises an alkyl oligosaccharide sulfate, or the physiologically acceptable salt thereof, as the effective component, wherein the oligosaccharide consists of 2 or more but 30 or less monosaccharides selected from a group consisting of glucose, galactose, mannose, talose, idose, altrose, allose, glucose, xylose, arabinose, rhamnose, fucose and fructose, and wherein the alkyl group is in either a straight or branched form having 22 carbon atoms or less.

The antiviral agent of the present invention expresses a very wide range of antiviral activity, effective against DNA viruses having DNA containing genetic information, such as Adenovirus, the herpes virus and the pox virus, and also effective against RNA viruses having RNA containing genetic information, such as Picorna virus, Toga virus, Orthomyxo virus, Paramyxo virus, Retrovirus and Corona virus. The antiviral agent of the present invention is also effective against viruses having envelopes, such as the herpes virus, Pox virus, Toga virus, Orthomyxo virus, Paramyxo virus, Retrovirus and Corona virus, and also effective against viruses having no envelope, such as Adenovirus and Picorna virus. The antiviral agent of the present invention is also effective against diseases caused simultaneously by two or more of these viruses.

Still more particularly, the antiviral agent of the present invention is effective in the prevention and treatment of the following diseases: pharyngitis, colds, conjunctivitus and diarrhea, which are caused by Adeno virus, a DNA virus having no envelope; inflammation of the cornea, chicken pox, and herpes zoster, labial and genital herpes, caused by the herpes virus, a DNA virus having an envelope; diseases caused by Picorna virus, an RNA virus having no envelope, such as infantile paralysis caused by polio virus; hand foot and mouth disease, myocardial, pericarditis, eruption and upper respiratory tract inflammation caused by Coxsackie virus; and colds caused by Rhinovirus; small pox, which is caused by Pox virus, a DNA virus having an envelope; Japanese encephalitis and rubella, which are caused by Toga virus, an RNA virus having an envelope, influenza, which is caused by Orthomyxo virus, an RNA virus having an envelope; bronchitis, the measles and pneumonia, which are caused by Paramyxo virus, an RNA virus having an envelope; AIDS, which is caused by HIV, an RNA retrovirus an envelope; and colds, which are caused by Corona virus, an RNA virus having an envelope.

As described above, the sulfated alkyl oligosaccharide of the present invention has antiviral activity against a variety of viruses, including the AIDS virus (HIV). By contrast, sulfated polysaccharide known for its activity against the AIDS virus has no recognized activity against a variety of viruses, except for the AIDS virus, and in particular is powerless against viruses having no envelope.

The mechanisms involved in the functions of the alkyl oligosaccharide sulfate as the antiviral agent of the present invention are not yet fully understood. It is apparent, however, that one of the mechanisms is the inhibition of the viruses from being bound by the target cells, as is the case with polysaccharide sulfates. The presumed reasons for the functions of the antiviral agent of the present invention which differ from those of the conventional antiviral agent of polysaccharide sulfate are (a) improved accessibility to the active site due to its smaller molecular size, and (b) improved affinity for cell surfaces due to the presence of an alkyl group having an affinity for lipid in addition to hydrophilic groups such as sulfate and hydroxide.

The antiviral agent of the present invention has an antiviral activity against a variety of viruses, as described above, and is therefore useful for treating the aforementioned diseases caused by one or more of these viruses. More particularly, the antiviral agent of the present invention is highly effective against the AIDS virus. It should be noted that many people who have reduced immune capacity by suffering with AIDS or cancer suffer from infections or complex infections diseases, called opportunistic infections, which are caused by bacteria or viruses with which healthy people are rarely infected, and die from these diseases instead of from HIV directly. They therefore receive administrations of a number of drugs with two or more strong side effects, in addition to anti-AIDS drug, and they suffer more from these side effects. Moreover, some types of bacteria or viruses are resistant to conventional drugs. For example, the herpes virus is found in many AIDS patents, and for this reason they are given acyclovir as an anti-Herpes agent. There is already an acyclovir-resistant strain of the herpes virus, and anti-the herpes virus drugs of different mechanisms are in strong demand. The antiviral agent of the present invention has high activity, not only against the AIDS virus but also against a variety of other viruses with different functions and mechanisms from those of conventional antiviral agents, including acyclovir, and most suited for treatment of patients with notably reduced immune capacity, in particular AIDS patents.

For example, the alkyl oligosaccharide sulfate, containing three monosaccharides having a lactose structure shows a very high activity of possessing an $EC_{50}$ level (50% effective concentration) of 10 μg/ml or less against the AIDS virus in vitro. The agent with a chain of 4 to 5 monosaccharides has a still higher activity with an $EC_{50}$ level of 0.2 to 0.3 μg/ml. This is a very important phenomenon which is not found with the conventional polysaccharide sulfate. A similar phenomenon is observed with the β (1 → 3) saccharide; an alkyl oligosaccharide of laminaripentaoside sulfate which is β (1 → 3) oligosaccharide of 5 glucose molecules unit has a very high activity of 0.68 μg/ml as the $EC_{50}$ level. The alkyl oligosaccharide of β (1 → 3) oligoglucoside sulfate consisting of 5 to 18 saccharides has a still higher activity of 0.074 μg/ml as the $EC_{50}$ level. All of these are of low toxicity, 1000 μg/ml or more in cytotoxicity. Thus it has been confirmed that the alkyl oligosaccharide sulfate of the present invention is high in activity and low in toxicity. The alkyl oligosaccharide sulfate of the present invention has high anti-HIV virus activity and alkylation of the oligosaccharide sulfate increases its anti-HIV activity, roughly 100 times, as illustrated in COMPARATIVE EXPERIMENTAL

EXAMPLE 1.

Acute toxicity tests with mice, orally and singly administered with the antiviral agent of the present invention, have revealed that all of the mice administered with any compound described later in the preferred embodiments remained alive at a dose rate of 1.0 g/kg. It is thus considered that the $LD_{50}$ (lethal dose 50%) level of the compounds of the present invention, when administered orally, is 1 g/kg or higher. Its level of intravenous-injected GTS-3, as one of the representative compounds of the present invention, is 1.2 g/kg, and 700 mg/kg with intravenous-injected LMS-1. The toxicity tests with mice, therefore, indicate that the antiviral agent of the present invention is very low in toxicity.

The antiviral agent of the present invention can be made into any ordinary drug form, such as tablets, capsules, granules, spheres, solution, injection, syrup and so on, for oral or non-oral administration. The ordinary vehicles and additives for common medicines are also used for the present invention. Ordinary vehicles include water, physiological saline, alcohol, polyethylene glycol, glycerol ester, gelatin, carbohydrate magnesium stearate, and talc. The ordinary additives include antiseptics, sterilizers, lubricants, coatings, wetting agents, emulsifiers, coloring agents, masking flavors, and aromas.

The dosage rate of the antiviral agent of the present invention is preferably in a range between 0.1 and 200 mg/kg of body weight, and more preferably between 0.5 and 100 mg, several times a day, though this varies depending on dosage method, dosage number, patient condition, patient body weight, and progress of the disease. It is preferably administered 1 to 3 times a day, though this number also varies depending on the medicine type, number of dosages, patient conditions, patient body weight, and progress of the disease. The antiviral agent of the present invention may be injected into the vein continuously.

[PREFERRED EMBODIMENTS]

The present invention is described in greater detail by the following preferred embodiments, which by no means limit the present invention.

(REFERENCE SYNTHESIS EXAMPLE 1): Synthesis of dodecyl β-D-maltopentaoside

First, 0.55 g of sodium acetate and 10 ml of acetic anhydride were heated in a flask, to which 1.003 g of D-maltopentaose was added little by little, and the mixture was heated under reflux for 2 hours. The reaction mixture was poured on 100 g of ice, and the reaction products were extracted with ethyl acetate. The ethyl acetate layer was dehydrated with anhydrous sodium sulfate and then concentrated to produce syrup. It was crystallized with ethyl acetate/hexane to produce peracetyl maltopentaose. The yield was 82%, and its α/β ratio, as determined by nuclear magnetic resonance spectral analysis, was 19/81.

Next, 500 mg of the peracetyl maltopentaose prepared above and 60 mg of n-dodecanol were dissolved in 20 ml of dichloromethane in a 50 ml three-necked flask, to which 1 mmol of tin tetrachloride was added and allowed to react at room temperature for 40 hours in a flow of nitrogen. The reaction mixture was poured into sodium bicarbonate solution, filtered by a celite-coated funnel, extracted with methylene chloride, dehydrated with anhydrous sodium sulfate, and concentrated. The residue was further refined by column chromatography (silica gel, hexane/ethyl acetate), and the fraction of Rf = 0.72 was collected by silica gel thin-layer chromatography (ethyl acetate/hexane = 3/1 by volume) and concentrated. The product obtained weighed 0.265 g (yield: 49%) and the melting point was 86° C to 90° C.

The product was dodecyl peracetyl-β-D-maltopentaoside, as identified by the nuclear magnetic resonance spectral analysis, described below:
Nuclear magnetic resonance spectra (proton)
Chemical shifts and coupling constants of the ring protons at positions 1 and 2 of saccharide bonded to alcohol:
Position 1: 4.51 ppm, J1, 2 = 8. 0 Hz
Position 2: 4.80 ppm, J2, 3 = 9. 6 Hz
Specific rotation: $[\alpha]_D$ = +106.3° (c = 1.0/chloroform) (25° C)

Next, 238 mg of the peracetate of glycoside prepared above, was dissolved in 10 ml of methanol, to which 4.3 ml of 0.1N sodium methoxide methanol solution was added at room temperature. The mixture was stirred for 2.5 hours and treated with an ion-exchanging resin to remove sodium ions. The methanol solution was concentrated and solidified after the ion-exchanging resin was removed by filtration in order to prepare 138 mg of the target product.
Specific rotation: $[\alpha]_D$ = +108.0° (c = 1.0/methanol) (25° C)
Melting point: 197.4 to 212.7° C

(COMPOUND EXAMPLE 1): Sulfation of dodecyl β-D-maltopentaoside (Sample MPS-1)

79 mg of dodecyl β-D-maltopentaoside, prepared by a method similar to that used for REFERENCE SYNTHESIS EXAMPLE 1, was dissolved in 10 ml of dehydrated dimethyl sulfoxide, to which 639 mg of piperidine sulfonic acid was added, and the mixture was stirred at 80° C for 1.5 hours. The mixture was then cooled to room temperature, to which 3.3 ml of deionized water was added, and stirred for 30 min. It was adjusted to a pH level of 7 to 8 with barium hydroxide, and the solids were separated centrifugally therefrom and allowed to pass through a Na ion-exchange column to produce sodium salts. The effluent was concentrated to 11 g and poured into 350 ml of acetone, and the precipitates formed were collected centrifugally, washed with acetone 3 times, and freeze-dried to remove water in order to produce 163 mg of the target product. This product was analyzed to have a sulfation index of 1.8 (sulfation ratio: 56.3%).
Specific rotation: $[\alpha]_D$ = +34.1° (c = 1.0/$H_2O$) (27° C)
Nuclear magnetic resonance spectra (proton) ($D_2O$) (ppm)

| | |
|---|---|
| 0.85 | alkyl methyl |
| 1.1 to 1.8 | alkyl methylene |
| 3.5 to 5.4 | hydrogen atoms in the saccharide structure, |

methylene at the terminal of oxygen, DOH

(COMPOUND EXAMPLE 2): Sulfation of dodecyl β-D-maltopentaoside (sample MPS-2)

100 mg of dodecyl β-D-maltopentaoside, prepared by a method similar to that used for REFERENCE SYNTHESIS EXAMPLE 1, was dissolved in 5 ml of dehydrated dimethyl sulfoxide, to which 265 mg of piperidine sulfonic acid was added, and the mixture was stirred at 80° C for 1.5 hours. The mixture was then cooled to room temperature, to which 4.0 ml of deionized water was added, and stirred for 30 min. It was adjusted to a pH level of 7 to 8 with barium hydroxide, and the solids were separated centrifugally therefrom and allowed to pass through a Na ion-exchanging column to produce sodium salts. The effluent was concentrated to approximately 7 g and poured in 350 ml of acetone, and the precipitates formed were collected centrifugally, washed with acetone 3 times and freeze-dried to remove water in order to produce 153 mg of the target product. This product was analyzed to have a sulfation index of 2.1 (sulfation ratio: 65.6%).

(REFERENCE SYNTHESIS EXAMPLE 2): Synthesis of dodecyl α-D-maltopentaoside

In the synthesis of dodecyl peracetyl β-D-maltopentaoside, a fraction of Rf = 0.54 was collected by silica gel thin-layer chromatography. The product was 89 mg in weight (yield: 19%), and its melting point was 82.1° C to 93.5° C.

Nuclear magnetic resonance spectra
Chemical shift and coupling constant of the ring protons at position 1 of saccharide bonded to alcohol:
Position 1: 4.83 ppm. J1, 2 = 4.0 Hz
Specific rotation: $[\alpha]_D$ = +123.2° (c = 1.0/chloroform) (25°C)

Next, dodecyl D-maltopentaoside was synthesized from 4.5 g of D-maltopentaose by a method similar to that for REFERENCE SYNTHESIS EXAMPLE 1. It was refined by column chromatography to produce 400 mg of the α-isomer, 194 mg of which was dissolved in 30 ml of methanol, to which 3.6 ml of 0.1N sodium methoxide methanol solution was added at room temperature. This mixture was stirred for 2.5 hours and treated with an ion-exchanging resin to remove sodium ions. Methanol was concentrated and solidified after the ion-exchanging resin was removed by filtration to prepare 116 mg of the crude product. It was then recrystallized from methanol/acetone to prepare 84 mg of the target product.
Specific rotation: $[\alpha]_D$ = +130.4° (c = 1.0/methanol) (25°C)
Melting point: 176 to 194°C

(COMPOUND EXAMPLE 3): Sulfation of dodecyl α-D-maltopentaoside (Sample MPS-3)

108 mg of dodecyl α-D-maltopentaoside, prepared by a method similar to that used for REFERENCE SYNTHESIS EXAMPLE 2, was dissolved in 20 ml of dehydrated dimethyl sulfoxide, to which 859 mg of piperidine sulfonic acid was added, and the mixture was stirred at 80°C for 1.5 hours. It was then cooled to room temperature, to which 5.5 ml of deionized water was added, and the mixture was stirred for 30 min. It was adjusted to a pH level of 7 to 8 with barium hydroxide, and the solids were separated centrifugally therefrom. The solution was allowed to pass through a Na ion-exchanging column to produce sodium salts. The effluent was concentrated to 18 g and poured into 350 ml of acetone, and the precipitates formed were collected by centrifugation. The solution was washed with acetone 3 times and freeze-dried to remove water to produce 228 mg of the target product. This product was analyzed to have a sulfation index of 2.8 (sulfation ratio: 87.5%).
Specific rotation: $[\alpha]_D$ = +53.0° (c = 1.0/$H_2O$) (25°C)
Nuclear magnetic resonance spectra (proton) ($D_2O$) (ppm)

| | |
|---|---|
| 0.85 | alkyl methyl |
| 1.1 to 1.8 | alkyl methylene |
| 3.5 to 5.5 | hydrogen atoms in the saccharide structure, |

methylene at the terminal of oxygen, DOH

(REFERENCE SYNTHESIS EXAMPLE 3): Synthesis of dodecyl β-D-laminaripentaoside

2.36 g of anhydrous sodium acetate and 80 ml of acetic anhydride were heated to around the boiling point in a 100 ml three-necked flask equipped with a condenser, thermometer, and solid supply.

4.0 g of laminaripentaose was added to the above flask little by little, and the mixture was heated under reflux for 1 hour. The reaction mixture was poured on 250 g of ice, stirred for 3 hours, extracted with chloroform, and washed with sodium bicarbonate solution and saturated sodium chloride solution. The syrup-like product was dissolved in ethanol after the solvent was removed, decolored by activated charcoal, and crystallized. It was then recrystallized from ethanol to produce 5.52 g of the crystals. Its α/β ratio, determined by the nuclear magnetic resonance spectral analysis, as was the case with REFERENCE SYNTHESIS EXAMPLE 1, was 22/78.

Next, 568 mg of peracetyl laminaripentaose prepared above and 83 mg of n-dodecanol were dissolved in 20 ml of dichloromethane in a 50 ml three-necked flask in a manner similar to that for REFERENCE SYNTHESIS EXAMPLE 1, to which 0.043 ml of tin tetrachloride was added, to allow them to react at room temperature for 4 hours in a flow of nitrogen. The reaction mixture was poured into sodium bicarbonate solution, filtered by a celite-coated funnel, extracted with dichloromethane, dehydrated with anhydrous sodium sulfate, and concentrated to dryness. It was further refined by column chromatography (silica gel, hexane/ethyl acetate), and the fraction of Rf = 0.72 was collected by silica gel, thin-layer chromatography (ethyl acetate/hexane = 3/1 by volume), and concentrated. The product was 229 mg in weight (yield: 37%).
Specific rotation: $[\alpha]_D$ = -52.9° (c = 1.0/chloroform) (25°C)
Melting point: 94.1 to 101.3°C

Next, the above product was deacetylated with a sodium methoxide/methanol solution to produce 94 mg of the target product from 164 mg of the acetate.
Specific rotation: $[\alpha]_D$ = -12.5° (c = 1.0/methanol) (40°C)
Melting point: 180 to 195°C

(COMPOUND EXAMPLE 4): Synthesis of sodium salt of sulfated dodecyl β-D-laminarpentaoside (Sample LPS-1)

185 mg of the target product was prepared from 102 mg of dodecyl β-D-laminaripentaoside, prepared in REFERENCE SYNTHESIS EXAMPLE 3, 877 mg of piperidine sulfonic acid and 10 ml of dimethyl sulfoxide, in a manner similar to that for COMPOUND EXAMPLE 1. It was analyzed to have a sulfation index of 2.5 (sulfation ratio: 78.1%).
Specific rotation: $[\alpha]_D$ = -7.9° (c = 1.0/$H_2O$ ) (27°C)
Nuclear magnetic resonance spectra (proton) ($D_2O$) (ppm)

| | |
|---|---|
| 0.85 | alkyl methyl |
| 1.1 to 1.8 | alkyl methylene |
| 3.5 to 5.5 | hydrogen atoms in the saccharide structure, |

methylene at the terminal of oxygen, DOH

(COMPOUND EXAMPLE 5): Synthesis of sodium salt of dodecyl β-D-laminaripentaoside sulfate (Sample LPS-1-1)

100 mg of dodecyl β-D-laminaripentaoside, prepared by a method similar to that used for REFERENCE SYNTHESIS EXAMPLE 3, was dissolved in 10 ml of dehydrated pyridine, to which 306 mg of sulfur trioxide/pyridine complex was added, and the mixture was stirred at 70°C for 1 hour. The mixture was then allowed to stand for cooling, and the pyridine layer as the supernatant liquor was separated from the precipitates by decantation. The precipitates were washed with pyridine and dried to prepare 327 mg of the residue. The residue was dissolved in water, adjusted to a pH level of 9.5 with a 2.5N sodium hydroxide solution, and refined by a "Maikuroashiraiza" (a small-scale desalter equipped with an AC110-10 membrane, supplied by Asahi Chemical Industries) to remove inorganic salts. "Maikuroashiraiza" precipitates were separated by adding acetone to the Maikuroashiraiza-treated solution. And the precipitates thus formed were dissolved in a small quantity of water and freeze-dried to prepare 254 mg of the target product. This product was analyzed to have a sulfation index of 3.0 (sulfation ratio: 93.8%).
Specific rotation: $[\alpha]_D$ = 6.2° (c = 1.0/$H_2O$ ) (27°C)
Nuclear magnetic resonance spectra (proton) ($D_2O$) (ppm)

| | |
|---|---|
| 0.85 | alkyl methyl |
| 1.1 to 1.8 | alkyl methylene |
| 3.5 to 5.5 | hydrogen atoms in the saccharide structure, |

methylene at the terminal of oxygen, DOH
The major spectral absorption levels of the infrared absorption spectra (KBr): 2960, 1640, 1240, 1150, 1000, 810 and 630 $cm^{-1}$

(COMPOUND EXAMPLE 6): Synthesis of sodium salt of n-hexadecyl β-laminaripentaoside sulfate (Sample LPS-2)

β-laminaripentaose was etherified with n-hecadecanol as alcohol and deacetylated, to prepare n-hexadecyl β-laminaripentaoside, in a manner similar to that for REFERENCE SYNTHESIS EXAMPLE 1. It was then sulfated, in a manner similar to that for COMPOUND EXAMPLE 4, to prepare the target product which is analyzed to have a sulfation index of 2.0 (sulfation ratio: 62.5%).
Specific rotation: $[\alpha]_D$ = -5.4° (c = 0.25/$H_2O$ ) (25°C)

(COMPOUND EXAMPLE 7): Synthesis of sodium salt of n-decyl β-laminaripentaoside sulfate (Sample LPS-3)

β-laminaripentaoside was etherified with n-decanol as alcohol and deacetylated, to prepare the target product, in a manner similar to that for COMPOUND EXAMPLE 6, and was analyzed to have a sulfation index of 1.8 (sulfation ratio: 56.3%).
Specific rotation: $[\alpha]_D$ = -6.2° (c = 0.25/$H_2O$) (25°C)

(COMPOUND EXAMPLE 8): Synthesis of sodium salt of dodecyl 4-galactosyl β-D-lactoside sulfate (Sodium salt of β-D-galactosyl (1 → 4)-β-D-galactosyl (1 → 4)-β-D-glucoside sulfate) (Sample GTS-1)

0.551 g of dodecyl 4-galactosyl-β-D-lactoside, prepared from 4-galactosyl-β-D-lactose in a manner similar to that used for REFERENCE SYNTHESIS EXAMPLE 1, was dissolved in 38 ml of pyridine, and the mixture was heated to 81°C. 3.64 g of sulfur trioxide/pyridine complex was added, and the components were allowed to react for 1.5 hours. The reaction mixture was cooled to room temperature, to which 11 ml of ion-exchanged water was added, and the mixture was stirred for 1.5 hours. The mixture was then adjusted to a pH level of 7.8 to 8.0 with barium hydroxide. The solution was then centrifuged to separate the precipitates, and passed through a Na type ion-exchanging column packed with Amberlite (IR-120B). The effluent was concentrated almost completely under a vacuum to distill off the solvent. It was then dissolved in approximately 10 g of ion-exchanged water, and poured into 250 ml of acetone to precipitate the sulfates. The precipitates were separated by centrifugation, and the precipitates were washed with acetone and freeze-dried to produce 0.72 g of the target product. This product was analyzed to have a sulfation index of 2.6 (sulfation ratio: 78.0%). Specific rotation: $[\alpha]_D$ = +26.9° (c = 0.58/$H_2$) (27°C)
The major spectral absorption levels of the infrared absorption spectra (KBr): 3460, 2930, 1640, 1240, 1020, 820 and 580 $cm^{-1}$

(COMPOUND EXAMPLE 9): Synthesis of sodium salt of dodecyl 4-galactosyl-β-D-lactoside sulfate (Sample GTS-2)

0.5 g of dodecyl 4-galactosyl-β-D-lactoside was dissolved in 38 ml of pyridine, and the mixture was heated to 85°C, in a manner similar to that for COMPOUND EXAMPLE 8. The same reaction and after-treatment procedures as used for COMPOUND EXAMPLE 8 were repeated hereafter, to produce 0.68 g of the target product. This product was analyzed to have a sulfation index of 2.8 (sulfation ratio: 84.0%).
Specific rotation: $[\alpha]_D$ = +24.5° (c = 0.51/$H_2O$) (27°C)

(COMPOUND EXAMPLE 10): Synthesis of sodium salt of dodecyl oligo-β (1 → 4)-galactosyl-β (1 → 4) glucoside sulfate (Sample GTS-3)

The oligosaccharide in which glucose is β-bonded at position 4 to the reduced terminal of the β (1 → 4)-type galacto-oligosaccharide (saccharide chain: 3 to 4) was peracetylated in a manner similar to that for REFERENCE SYNTHESIS EXAMPLE 1, converted into glycoside with dodecanol, and then deacetylated with sodium methoxide/methanol. The yield was 90%.
Specific rotation: $[\alpha]_D$ = +46.8° (c = -0.52/$H_2O$) (27°C)

It was sulfated in a manner similar to that for COMPOUND EXAMPLE 8 to produce 0.61 g of the target product from 0.51 g of the starting material. This product was analyzed to have a sulfation index of 3.0 (sulfation ratio: 93.1%).
Specific rotation: $[\alpha]_D$ = +24.5° (c = 0.52/$H_2O$) (27°C)
Nuclear magnetic resonance spectra (proton) ($D_2O$) (ppm)

| | |
|---|---|
| 0.85 | alkyl methyl |
| 1.1 to 1.8 | alkyl methylene |
| 3.3 to 5.3 | hydrogen atoms in the saccharide structure, |

methylene at the terminal of oxygen, DOH
The major spectral absorption levels of the infrared absorption spectra (KBr): 3460, 2930, 1630, 1240, 1010, 820 and 580 $cm^{-1}$

(COMPOUND EXAMPLE 11): Synthesis of sodium salt of octadecyl oligogalactosyl-β (1 → 4) glucoside sulfate (Sample GTS-4)

The oligosaccharide in which glucose is β-bonded at position 4 to the reduced terminal of the β (1 → 4)-type galacto-oligosaccharide (saccharide chain: 3 to 4) was treated in a manner similar to that for COMPOUND EXAMPLE 11 with octadecanol as the alcohol, and then treated hereafter in the same manner as that for COMPOUND EXAMPLE 10, to prepare glycoside.
Specific rotation: $[\alpha]_D$ = +49.9° (c = 0.50/MeOH) (27°C)

The compound was then sulfated, in a manner similar to that for COMPOUND EXAMPLE 10, to produce 0.44 g of the target product from 0.52 g of the starting material.

This product was analyzed to have a sulfation index of 2.2 (sulfation ratio: 68.3%).
Specific rotation: $[\alpha]_D$ = +23.1° (c = 0.58/$H_2O$) (27°C)
Nuclear magnetic resonance spectra (proton) ($D_2O$) (ppm)

| | |
|---|---|
| 0.85 | alkyl methyl |
| 1.1 to 1.8 | alkyl methylene |
| 3.3 to 5.3 | hydrogen atoms in the saccharide structure, |

methylene at the terminal of oxygen, DOH

The major spectral absorption levels of the infrared absorption spectra (KBr): 3460, 2930, 1630, 1240, 1010, 820 and 580 $cm^{-1}$

(COMPOUND EXAMPLE 12): Synthesis of sodium salt of laminarioligosaccharide dodecanol sulfate (Sample LMS-1)

3.17 g of β (1 → 3) oligosaccharide (average molecular weight: 1800) was acetylated with acetic anhydride and sodium acetate by the ordinary method to prepare 4.32 g of peracetylate. 2.02 g of peracetylate prepared above was reacted in the presence of 0.05 g of phosphotungstate as the catalyst at 102° C. It was treated in a manner similar to that for REFERENCE SYNTHESIS EXAMPLE 3 to prepare 1.7 g of dodecyl ether (acetylated dodecanol-glycoside), then deacetylated with sodium methoxide/methanol to prepare 0.9 g of glycoside. Specific rotation: $[\alpha]_D$ = +26.5° (c = 0.52/$H_2O$) (27°C)

It was then sulfated in a manner similar to that for COMPOUND EXAMPLE 8 to prepare 0.40 g of the target product from 0.315 g of the starting material.

This product was analyzed to have a sulfation index of 2.6 (sulfation rate: 84.1%)
Specific rotation: $[\alpha]_D$ = +9.9° (c = 0.51/$H_2O$) (27°C)
Nuclear magnetic resonance spectra (proton) ($D_2O$) (ppm)

| | |
|---|---|
| 0.85 | alkyl methyl |
| 1.1 to 1.8 | alkyl methylene |
| 3.3 to 5.4 | hydrogen atoms in the saccharide structure, |

methylene at the terminal of oxygen, DOH
The major spectral absorption levels of the infrared absorption spectra (KBr): 3460, 2930, 1630, 1240, 1000, 800 and 590 $cm^{-1}$

(COMPOUND EXAMPLE 13): Synthesis of sodium salt of octadecyl maltotrioside sulfate (Sample MTS-1)

Octadecyl maltotrioside was prepared from maltotriose in a manner similar to that for REFERENCE SYNTHESIS EXAMPLE 1.
Specific rotation: $[\alpha]_D$ = +85.7° (c = 1.0/MeOH) (27°C)

It was then sulfated with piperidine sulfonic acid/dimethyl sulfoxide to prepare 123 mg of the target product from 0.098 mg of the starting material.
Specific rotation: $[\alpha]_D$ = +50.3° (c = 1.0/$H_2O$) (27°C)

This product was analyzed to have a sulfation index of 2.0 (sulfation rate: 60.0%)

(COMPOUND EXAMPLE 14): Synthesis of sodium salt of octadecyl maltoheptaoside sulfate (Sample MHS-1)

Octadecyl maltoheptaoside was prepared from maltoheptaose in a manner similar to that for REFERENCE SYNTHESIS EXAMPLE 1.
Specific rotation: $[\alpha]_D$ = +141.7° (c = 1.0/MeOH) (27°C)

It was then sulfated with piperidine sulfonic acid/dimethyl sulfoxide to prepare 115 mg of the target product from 0.080 g of the starting material.
Specific rotation: $[\alpha]_D$ = +78.8° (c = 1.0/$H_2O$) (27°C)

This product was analyzed to have a sulfation index of 1.0 (sulfation rate: 31.8%).

(COMPOUND EXAMPLE 15): Synthesis of sodium salt of dodecyl maltohexaoside sulfate (Sample MHS-2)

Dodecyl maltohexaoside was prepared from maltohexaose, in a manner similar to that for REFERENCE SYNTHESIS EXAMPLE 1.

It was sulfated with piperidine sulfonic acid/dimethyl sulfoxide, to prepare 118 mg of the target product from 80 mg of the starting material.

The product was analyzed to have a sulfation index of 2.8 (sulfation rate: 88.4%).

(COMPOUND EXAMPLE 16): Synthesis of sodium salt of octadecyl maltohexaoside sulfate (Sample MHS-3)

Octadecyl maltohexaoside was prepared from maltohexaose in a manner similar to that for REFERENCE SYNTHESIS EXAMPLE 1.

It was sulfated with piperidine sulfonic acid/dimethyl sulfoxide to prepare 110 mg of the target product from 75 mg of the starting material. This product was analyzed to have a sulfation index of 2.9 (sulfation rate: 91%)

(COMPARATIVE EXAMPLE 1).

Laminaripentaose, as used in REFERENCE SYNTHESIS EXAMPLE 1, was directly sulfated with a sulfur trioxide/pyridine complex in a manner similar to that for COMPOUND EXAMPLE 5. It was analyzed to have a sulfation index of 2.9 (sulfation rate: 85.3%).

(COMPARATIVE EXAMPLE 2)

4-galactosyl-β-D-lactose, as used in REFERENCE SYNTHESIS EXAMPLE 8, was directly sulfated with a sulfur trioxide/pyridine complex in a manner similar to that for COMPOUND EXAMPLE 5. It was analyzed to have a sulfation index of 2.9 (sulfation rate: 79.1%).

(COMPARATIVE EXAMPLE 3)

500 mg of 4-galactosyl-β-D-lactose, as used in COMPOUND EXAMPLE 8, was dissolved in 10 ml of anhydrous dimethyl sulfoxide, to which 0.43 ml of 1-iodine octane (0.22 equivalents to the hydroxide group in the starting saccharide) and 88 mg of potassium hydroxide powder were added, and they were allowed to react at 50°C for 8 hours under a flow of nitrogen. The reaction mixture was then sulfated with 2.81 g of sulfur trioxide/pyridine complex for 3 hours. Following this, the solvent was concentrated under a vacuum. The residue was dissolved in water, neutralized with a 2N sodium hydroxide solution, and refined by ultrafiltration. The product was 1.10 g in weight and brown in color. The alkylation rate of the hydroxide group was 10.9% as estimated from the ratio of protons in alkyl methyl group to that in the saccharide, determined by the nuclear magnetic resonance analysis. The product was analyzed to have a sulfation index of 2.5 (sulfation rate: 75.0%).

The alkyl group thus synthesized, as expected from the method by which it was synthesized, was found to be bonded to the hydroxide groups other than those at the reduced terminals of the saccharide, and two or more alkyl groups were found in the compound.

The compounds prepared by EXAMPLES were tested for their antiviral activity.

(ANTIVIRAL ACTIVITY TEST EXAMPLE 1)

Samples: MPS-1, MPS-2, MPS-3 and LPS-1
Cell and Virus: MT-4 Cell, MOLT-4 Cell
HTLV as one HIV strain was prepared from the supernatant of the HIV-infected cells. The titer for the virus

preparation (6 x $10^5$ plaque formation units/ml) was measured by the plaque assay method using MT-4 cells.

(Measurement of anti-HIV activity)

The anti-HIV activity of each sample was evaluated by the HIV-induced cytopathic effect (CPE) and the expression of the virus-specific antigen.

The MT-4 cells were mixed with HIV at 0.002MOI (multiplicity of infection), to be cultured at 37°C for 60 min. The virus-absorbing cells were washed and adjusted at 3 x $10^5$ cells/ml (the culture medium was RPMI1640 which supplemented with 10% heat-inactivated fetal calf serum and antibiotic). The suspension of the infected cells was cultured in the presence of the sample, and then cultured in a $CO_2$-containing atmosphere for 3 days. Then, the medium was replaced with the one containing the same quantity of the sample. Percentages of the viable cells and virus antigen positive cells were counted by the trypan blue exclusion method and indirect fluorescent antibody (IF) methods, respectively.

The test results are given in Tables 1 through 8, where the units for concentration, mock-infected cell count, infected cell count, percentage of HIV-antigen positive cell are $\mu$g/ml, x $10^4$ cells/ml, and x $10^4$ cells/ml, %, respectively. These results are also illustrated in Figures 1 through 8.

The results shown in Table 1 (for Sample MPS-1 on the third day) and in Table 2 (for Sample MPS-1 on the 6th day) have indicated that the HIV-infected cell count and the mock-infected cell count are almost the same at a concentration of 100 $\mu$g/ml. Table 3 (for Sample MPS-2 on the third day) and Table 4 (for Sample MPS-2 on the 6th day) show the results similar to those shown in Tables 1 and 2. Furthermore, Table 5 (for Sample MPS-3 on the third day), Table 6 (for Sample MPS-3 on the 6th day), Table 7 (for Sample LPS-1 on the third day), and Table 8 (for Sample LPS-1 on the 6th day) also show similar results. It has therefore been confirmed that these samples function effectively as anti-HIV agents.

Table 1

| MPS-1 (On the 3rd day) | | | |
|---|---|---|---|
| Concentration | Mock-infected cell count | HIV-infected cell count | Percentage of HIV-antigen positive cell |
| 0 | 176 | 119 | 11 |
| 25 | | 134 | 5 |
| 50 | 172 | 126 | 1 |
| 100 | 161 | 129 | < 1 |
| 200 | 142 | | < 1 |
| 500 | | | < 1 |

Table 2

| MPS-1 (On the 6th day) | | | |
|---|---|---|---|
| Concentration | Mock-infected cell count | HIV-infected cell count | Percentage of HIV-antigen positive cell |
| 0 | 175 | 16 | > 90 |
| 25 | 167 | 47 | > 90 |
| 50 | 176 | 120 | 25 |
| 100 | 115 | 101 | < 1 |
| 200 | 106 | | < 1 |

Table 3

| MPS-2 (On the 3rd day) | | | |
|---|---|---|---|
| Concentration | Mock-infected cell count | HIV-infected cell count | Percentage of HIV-antigen positive cell |
| 0 | 176 | 119 | 11 |
| 25 | | 144 | 5 |
| 50 | 182 | 132 | 2 |
| 100 | 184 | 137 | 1 |
| 200 | 180 | | < 1 |
| 500 | 126 | | < 1 |

Table 4

| MPS-2 (On the 6th day) | | | |
|---|---|---|---|
| Concentration | Mock-infected cell count | HIV-infected cell count | Percentage of HIV-antigen positive cell |
| 0 | 175 | 16 | 90 |
| 25 | | 35 | 90 |
| 50 | 178 | 100 | 24 |
| 100 | 70 | 86 | < 1 |
| 200 | 73 | | < 1 |

Table 5

| MPS-3 (On the 3rd day) | | | |
|---|---|---|---|
| Concentration | Mock-infected cell count | HIV-infected cell count | Percentage of HIV-antigen positive cell |
| 0 | 176 | 119 | 11 |
| 25 | | 127 | 3 |
| 50 | 171 | 133 | < 1 |
| 100 | 186 | 122 | < 1 |
| 200 | 142 | | < 1 |
| 500 | 83 | | < 1 |

Table 6

| MPS-3 (On the 6th day) | | | |
|---|---|---|---|
| Concentration | Mock-infected cell count | HIV-infected cell count | Percentage of HIV-antigen positive cell |
| 0 | 175 | 16 | > 90 |
| 25 | 159 | 93 | > 90 |
| 50 | 166 | 106 | 5 |
| 100 | 165 | 123 | < 1 |
| 200 | 145 | | < 1 |

Table 7

| LPS-1 (On the 3rd day) | | | |
|---|---|---|---|
| Concentration | Mock-infected cell count | HIV-infected cell count | Percentage of HIV-antigen s positive cell |
| 0 | 176 | 119 | 11 |
| 25 | 151 | 133 | 3 |
| 50 | 149 | 128 | < 1 |
| 100 | 136 | 130 | < 1 |
| 200 | 120 | | < 1 |
| 500 | 79 | | < 1 |

Table 8

| LPS-1 (On the 6th day) | | | |
|---|---|---|---|
| Concentration | Mock-infected cell count | HIV-infected cell count | Percentage of HIV-antigen positive cell |
| 0 | 175 | 16 | > 90 |
| 25 | 145 | 88 | 20 |
| 50 | 149 | 150 | < 1 |
| 100 | 140 | 107 | 0 |
| 200 | 124 | | 0 |

(ANTIVIRAL ACTIVITY TEST 2)

Test Method: MMT (the tetrazolium assay) method
Cell: MT-4 Cell
Virus: HTLV-$III_B$ (Infection : 3 x $10^5$ plaque forming units (PFU)/ml)

MT-4 cells were suspended in a culture medium at 2 x $10^5$ cells/ml and infected with HIV-$III_B$ at a multiplicity of infection of 0.01. Immediately after infection, 100μl of cell suspension was placed into microtiter tray wells containing various concentrations of the test samples. At the same time, the mock-infected cells were also cultured together with the samples in order to assess the cell toxicity of the test samples to the MT-4 cell. They were cultured in a $CO_2$-gas incubator at 37° C for 5 days, and the number of viable cells were determined by the MTT method. Antiviral activity is expressed by $EC_{50}$ (50% effective concentration), the concentration at which the agent protects 50% of the cells from injury by HIV infection, and cell toxicity by $CC_{50}$ (50% cell toxicity concentration), the concentration at which 50% of the cells are injured by the agent. Selectivity is measured by a selectivity index (S.I), which is expressed by $CC_{50}/EC_{50}$ - (R. Pauwels, et al., J. of Biological Methods, vol. 20, 1988, pp. 309-321).

Samples LPS-2 and LPS-3 were evaluated by the above method for their anti-HIV activity. The results are given in Table 9.

The same method was used to determine anti-HIV activity of Samples GTS-1, GTS-2, GTS-3, GTS-4, LMS-1, MTS-1 and MHS-1. The results are given in Table 10. The table also gives the test results of 3'-azide-3'-deoxythymidine (AZT) as the existing anti-HIV agent for purposes of comparison.

Table 11 gives the results of LPS-1-1, MHS-2, MHS-3, and the samples prepared by COMPARATIVE EXAMPLES 1 through 3, evaluated by the same method. These test results have indicated that each compound of the present invention is useful as an anti-HIV agent, and that GTS-3, GTS-4 and LMS-1, in particular, are much more selective and hence better than AZT.

Table 9

| Test agent samples | $CC_{50}$ µg/ml | $EC_{50}$ µg/ml | SI |
|---|---|---|---|
| LPS-2 | = 471 | = 17 | = 27 |
| LPS-3 | > 1000 | = 67 | > 15 |

Table 10

| Test agent samples | $CC_{50}$ µg/ml | $EC_{50}$ µg/ml | SI |
|---|---|---|---|
| GTS-1 | 730 | 9.8 | 75 |
| GTS-2 | 858 | 8.6 | 94 |
| GTS-3 | > 2000 | 0.1886 | > 10604 |
| GTS-4 | > 2000 | 0.3241 | > 6170 |
| MTS-1 | 329 | 1) > 40 | > 1 |
| MHS-1 | 355 | 2) > 40 | > 1 |
| LMS-1 | > 2000 | 0.0738 | > 27100 |
| 3) AZT | 3.06 | 0.0019 | 1624 |

Notes for Table 10
1) 41% protection/40 µg/ml
2) 22% protection/40 µg/ml
3) Comparative data

Table 11

| Tested agent samples | $CC_{50}$ µg/ml | $EC_{50}$ µg/ml | SI |
|---|---|---|---|
| LPS-1-1 | > 1000 | 0.68 | > 1470 |
| MHS-2 | > 1000 | 3.91 | > 256 |
| MHS-3 | 580 | 10.36 | 56 |
| COMPARATIVE EXAMPLE 1 | > 1000 | 88.3 | > 12 |
| COMPARATIVE EXAMPLE 2 | > 1000 | > 1000 | > < 1 |
| COMPARATIVE EXAMPLE 3 | > 1000 | 861 | > 1 |

(ANTIVIRAL TEST EXAMPLE 3)

Antiviral activity against the herpes virus (HSV) (Herpes simplex virus) type 1 was evaluated.
Test Cell: Vero Cell
Test Method: Plaque reduction method

The tests were conducted under conditions similar to those used for Tests 1 and 2. The averaged results are given in Table 12.

As shown in Table 12, the $EC_{50}$ levels of Samples GTS-3 and LMS-1 are 18.8 and 16.7 µg/ml, respectively.

Table 12

| Tested agent samples (50% effective concentration) (µg/ml) | | |
|---|---|---|
| | GTS - 3 | LMS - 1 |
| Run 1<br>Run 2 | 25.9<br>11.7 | 9.1<br>24.2 |
| Average | 18.8 | 16.7 |
| Cell toxicity: 100 µg/ml | | |

(ANTIVIRAL TEST EXAMPLE 4)

Antiviral activity against Adenovirus type 3 was measured.
Test Cell: MKC-5 Cell
Test Method: A mono-layer, 24-well plate was infected with the virus at 100 to 200 CCID50/well, to which agent samples of varying concentrations were added immediately after the infection, and cultured at 37 ° C. The CPE was observed microscopically one week later. The results are given in Table 13.

As shown in Table 13, the $EC_{50}$ levels of Samples GTS-3 and LMS-1 are 20 and 20 to 100 µg/ml, respectively.

Table 13

| Concentration (μ/g/ml) | Tested agent samples | |
|---|---|---|
| | LMS - 1 | GTS - 3 |
| 500 | (-) | (-) |
| 100 | (-) | (±) |
| 20 | (+) | (+++) |
| Virus control | (+++) | |
| Cell toxicity | (-) | (-) |

(ANTIVIRAL TEST EXAMPLE 5)

Antiviral activity against Coxsackie virus B-3: Nancy strain was evaluated.

Test Cell: Vero Cell

Test Method: The cytopathic effect and virus production rate were measured 48 hours after inoculation.

The results are given in Tables 14 through 17, where each cell control shows a (-) rating.

Based on these results, the $EC_{50}$ levels of Samples LMS-1 and GTS-3 are judged to be 7.5 and 3.8 μg/ml, respectively.

Table 14: Degree of cytopathic effect and virus production rate 1 (48 hours after the virus inoculation, Vero Cell)

| LMS-1 concentration | Degree of CPE | Virus production rate (PFU) |
|---|---|---|
| 100 μg/ml | + | $2.0 \times 10^5$ |
| 10 | + + | $1.4 \times 10^6$ |
| 1 | + + + | $5.0 \times 10^6$ |
| 0.1 | + + + | $5.0 \times 10^6$ |
| Virus control | + + + | $4.0 \times 10^6$ |

Table 15: Degree of cytopathic effect and virus production rate 2 (48 hours after the virus inoculation, Vero Cell)

| LMS-1 concentration | Degree of CPE | Virus production rate (PFU) |
|---|---|---|
| 100 μg/ml | + | $1.0 \times 10^6$ |
| 10 | + + | $2.0 \times 10^6$ |
| 1 | + + + | $3.0 \times 10^6$ |
| 0.1 | + + + | $8.0 \times 10^6$ |
| Virus control | + + + | $4.0 \times 10^6$ |

Table 16: Degree of cytopathic effect and virus production rate 3 (48 hours after the virus inoculation, Vero Cell)

| GTS-1 concentration | Degree of CPE | Virus production rate (PFU) |
|---|---|---|
| 100 μg/ml | + | $5.0 \times 10^5$ |
| 10 | + + | $4.0 \times 10^6$ |
| 1 | + + | $6.0 \times 10^6$ |
| 0.1 | + + + | $8.0 \times 10^6$ |
| Virus control | + + + | $8.0 \times 10^6$ |

Table 17: Degree of cytopathic effect and virus production rate 4 (48 hours after the virus inoculation, Vero Cell)

| GTS-1 concentration | Degree of CPE | Virus production rate (PFU) |
|---|---|---|
| 100 μg/ml | + | $9.0 \times 10^5$ |
| 10 | + | $2.0 \times 10^6$ |
| 1 | + + | $4.0 \times 10^6$ |
| 0.1 | + + + | $1.1 \times 10^7$ |
| Virus control | + + + | $9.0 \times 10^6$ |

PRODUCTION EXAMPLES for the antiviral agent of the present invention are described below:

(ANTIVIRAL AGENT PRODUCTION EXAMPLE 1)

| | |
|---|---|
| GTS-3 (Compound prepared in COMPOUND EXAMPLE 10) | 50 mg |
| Starch | 30 mg |
| Lactose | 110 mg |
| Talc | 7 mg |
| Magnesium stearate | 3 mg |
| | 200 mg |

Compound GTS-3 was ground and mixed together with lactose and starch. Then, 10% of starch paste was added to the above mixture, which was stirred until granules were formed. The granules were dried and sieved, talc and magnesium stearate were added, and the mixture was processed by the ordinary method to prepare the 200 mg tablet.

(ANTIVIRAL AGENT PRODUCTION EXAMPLE 2)

| | |
|---|---|
| LMS-1 (Compound prepared in COMPOUND EXAMPLE 12) | 50 mg |
| Starch | 30 mg |
| Lactose | 110 mg |
| Talc | 7 mg |
| Magnesium stearate | 3 mg |
| | 200 mg |

The same procedure used in ANTIVIRAL AGENT PRODUCTION EXAMPLE 1 was repeated for Compound LMS-1 to prepare the 200 mg tablet.

(ANTIVIRAL AGENT PRODUCTION EXAMPLE 3)

| | |
|---|---|
| LPS-1 (Compound prepared in COMPOUND EXAMPLE 4) | 50 mg |
| Starch | 30 mg |
| Lactose | 110 mg |
| Talc | 7 mg |
| Magnesium stearate | 3 mg |
| | 200 mg |

The same procedure used in ANTIVIRAL AGENT PRODUCTION EXAMPLE 1 was repeated for Compound LPS-1 to prepare the 200 mg tablet.

(ANTIVIRAL AGENT PRODUCTION EXAMPLE 4)

Physiological saline solution that had been sterilized by an autoclave was added to 500 mg of GTS-3 (compound prepared in COMPOUND EXAMPLE 10) to a total volume of 10 ml to dissolve the compound therein. The mixture was then transferred into an ampule to prepare 10 ml of the medicine.

(ANTIVIRAL AGENT PRODUCTION EXAMPLE 5)

Physiological saline solution was added to 500 mg of LMS-1 (Compound prepared in COMPOUND EXAMPLE 12) to prepare 10 ml of the medicine using the same procedure as used for ANTIVIRAL AGENT PRODUCTION EXAMPLE 4.

(ANTIVIRAL AGENT PRODUCTION EXAMPLE 6)

Physiological saline solution was added to 600 mg of LPS-1 (compound prepared in COMPOUND EXAMPLE 4) to prepare 10 ml of the medicine using the same procedure as was used for ANTIVIRAL AGENT PRODUCTION EXAMPLE 4.

(ANTIVIRAL AGENT PRODUCTION EXAMPLE 7)

| | |
|---|---|
| LMS-1 (compound prepared in COMPOUND EXAMPLE 12)<br>Lactose<br>Starch<br>Magnesium stearate | 25 mg<br>50 mg<br>23 mg<br>2 mg |
| | 100 mg |

25 mg of Compound LMS-1 (compound prepared in COMPOUND EXAMPLE 12) was ground well and mixed together with lactose, starch and magnesium stearate. The mixture was then transferred into a capsule to prepare the capsulated medicine.

(ANTIVIRAL AGENT PRODUCTION EXAMPLE 8)

| | |
|---|---|
| GTS-3 (compound prepared in COMPOUND EXAMPLE 10)<br>Lactose<br>Starch<br>Magnesium stearate | 25 mg<br>50 mg<br>23 mg<br>2 mg |
| | 100 mg |

The same procedure as used for ANTIVIRAL AGENT PRODUCTION EXAMPLE 7 was repeated for Compound GTS-S (compound prepared in COMPOUND EXAMPLE 10), to prepare 100 mg of the capsulated medicine.

[POSSIBILITIES OF INDUSTRIAL UTILIZATION]

The present invention provides an antiviral agent which is low in toxicity and exhibits antiviral activity against a wide range of viruses, in particular an anti-HIV agent with a broad antiviral spectrum.

**Claims**

**1.** An alkyl oligosaccharide sulfate or a physiologically acceptable salt of the same, which is formed from a single monosaccharide bonded by glycoside bonds, wherein the hydrogen atom in the hydroxyl group at the position-1 carbon atom at the terminal of said oligosaccharide has been substituted with a straight or branched alkyl group, and at least 14.3% of the remaining hydroxyl group has been substituted with a sulfate group.

**2.** An alkyl oligosaccharide sulfate or a physiologically acceptable salt of the same, according to Claim 1, wherein said monosaccharide is selected from a group consisting of galactose, mannose, talose, idose, altrose, allose, glucose, xylose, arabinose, rhamnose, fucose, and fructose.

**3.** An alkyl oligosaccharide sulfate or a physiologically acceptable salt of the same, according to Claim 2, wherein said oligosaccharide consists of 2 or more of said monosaccharides but 30 or fewer of said monosaccharides.

**4.** An alkyl oligosaccharide sulfate or a physiologically acceptable salt of the same, which is formed from a combination of different types of monosaccharides bonded by glycoside bonds, wherein the hydrogen atom in the hydroxyl group at the position-1 carbon atom at the terminal of said oligosaccharide has been substituted with a straight or branched alkyl group, and at least 14.3% of the remaining hydroxyl group has been substituted with a sulfate group.

**5.** An alkyl oligosaccharide sulfate or a physiologically acceptable salt of the same, according to Claim 4, wherein said monosaccharide is selected from a group consisting of glucose, galactose, mannose, talose, idose, altrose, allose, glucose, xylose, arabinose, rhamnose, fucose, and fructose.

**6.** An alkyl oligosaccharide sulfate or a physiologically acceptable salt of the same, according to Claim 5, wherein said oligosaccharide consists of 2 or more of said monosaccharides but 30 or fewer of said monosaccharides.

**7.** An alkyl oligosaccharide sulfate or a physiologically acceptable salt of the same, according to Claim 4, wherein said oligosaccharide is galactose-based having a lactose structure characterized by galactose being bonded by the $\beta$ (1 → 4)-glycoside bond to the galactose portion in said lactose structure at position-4 and successively bonded by the $\beta$ (1 → 4) bonds to the terminal galactose portions at position-4.

**8.** An alkyl oligosaccharide sulfate or a physiologically acceptable salt of the same, according to Claim 7, wherein said oligosaccharide consists of 2 or more of said monosaccharides but 30 or fewer of said monosaccharides.

**9.** An alkyl oligosaccharide sulfate or a physiologically acceptable salt of the same, which is formed from glucose as the single constituent monosaccharide bonded by the $\beta$(1 → 3) glycoside bonds, wherein the hydrogen atom in the hydroxyl group at the position-1 carbon atom at the terminal of said oligosaccharide has been substituted with a straight or branched alkyl group, and at least 14.3% of the remaining hydroxyl group has been substituted with a sulfate group.

**10.** An alkyl oligosaccharide sulfate or a physiologically acceptable salt of the same, according to Claim 9, wherein said oligosaccharide consists of 2 or more of said monosaccharides but 30 or fewer of said monosaccharides.

**11.** An alkyl oligosaccharide sulfate or a physiologically acceptable salt of the same, according to Claims 1 through 10, wherein said alkyl group is either a straight or branched alkyl group, having 22 or fewer carbon atoms.

**12.** An antiviral agent that comprises, as the effective component, alkyl oligosaccharide sulfate or a physiologically acceptable salt of the same.

**13.** An antiviral agent according to Claim 12, exhibiting an antiviral activity against DNA viruses.

**14.** An antiviral agent according to Claim 12, exhibiting an antiviral activity against RNA viruses.

**15.** An antiviral agent according to Claim 12, exhibiting an antiviral activity against viruses having an envelope.

**16.** An antiviral agent according to Claim 12, exhibiting an antiviral activity against viruses having no envelope.

**17.** An antiviral agent according to Claim 12, exhibiting an antiviral activity against Retroviruses.

**18.** An antiviral agent according to Claim 12, exhibiting an antiviral activity against HIV.

**19.** An antiviral agent according to Claim 12, exhibiting an antiviral activity against Adenoviruses.

**20.** An antiviral agent according to Claim 12, exhibiting an antiviral activity against the herpes virus.

**21.** An antiviral agent according to Claim 12, exhibiting an antiviral activity against Coxsackie virus.

**22.** An antiviral agent that comprises, as the effective component, alkyl oligosaccharide sulfate or a physiologically acceptable salt of the same according to Claim 11.

**23.** An antiviral agent according to Claim 22, exhibiting an antiviral activity against DNA viruses.

**24.** An antiviral agent according to Claim 22, exhibiting an antiviral activity against RNA viruses.

**25.** An antiviral agent according to Claim 22, exhibiting an antiviral activity against viruses having an envelope.

**26.** An antiviral agent according to Claim 22, exhibiting an antiviral activity against viruses having no envelope.

**27.** An antiviral agent according to Claim 22, exhibiting an antiviral activity against Retroviruses.

**28.** An antiviral agent according to Claim 22, exhibiting an antiviral activity against HIV.

**29.** An antiviral agent according to Claim 22, exhibiting an antiviral activity against Adenoviruses.

**30.** An antiviral agent according to Claim 22, exhibiting an antiviral activity against the herpes virus.

**31.** An antiviral agent according to Claim 22, exhibiting an antiviral activity against Coxsackie virus.

FIG.1

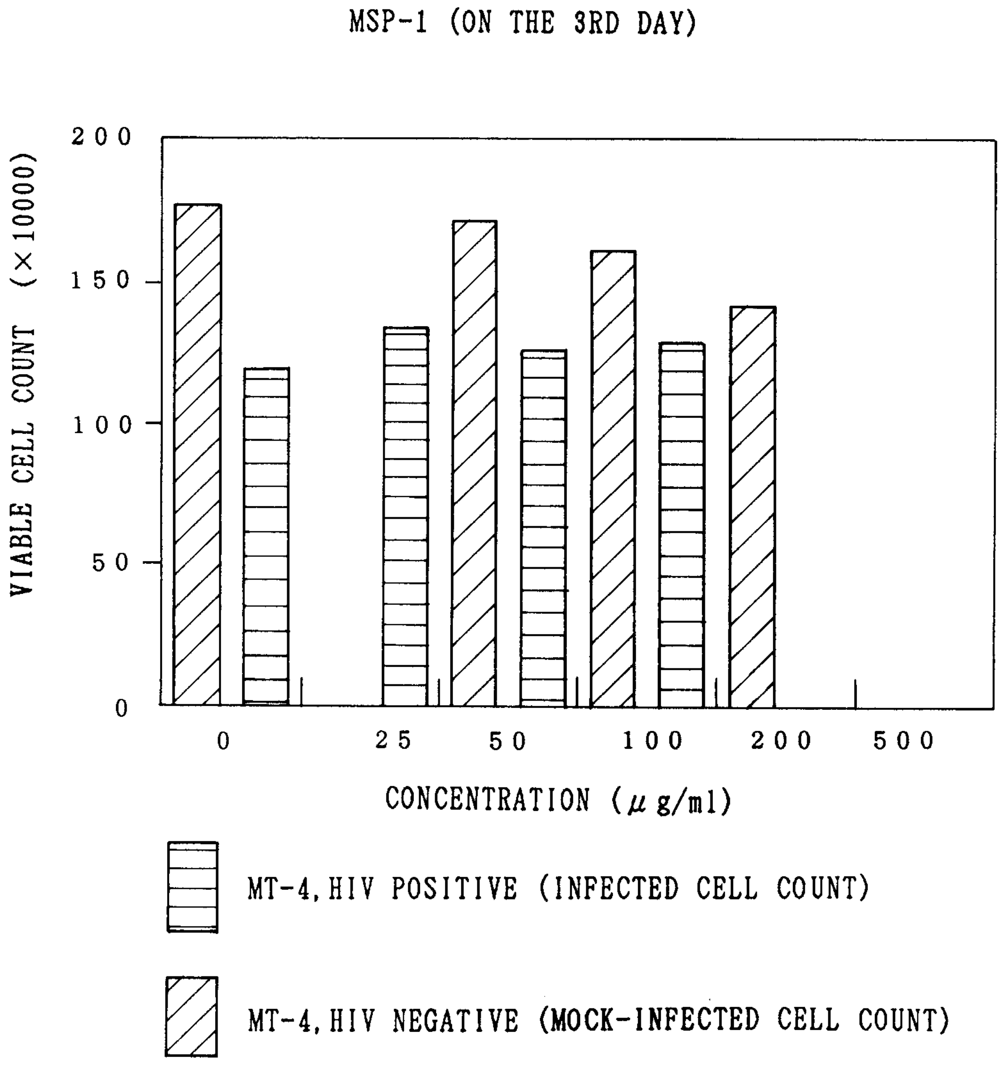
MSP-1 (ON THE 3RD DAY)
VIABLE CELL COUNT (×10000)
200
150
100
50
0
0
25
50
100
200
500
CONCENTRATION (μg/ml)
MT-4, HIV POSITIVE (INFECTED CELL COUNT)
MT-4, HIV NEGATIVE (MOCK-INFECTED CELL COUNT)

FIG.2

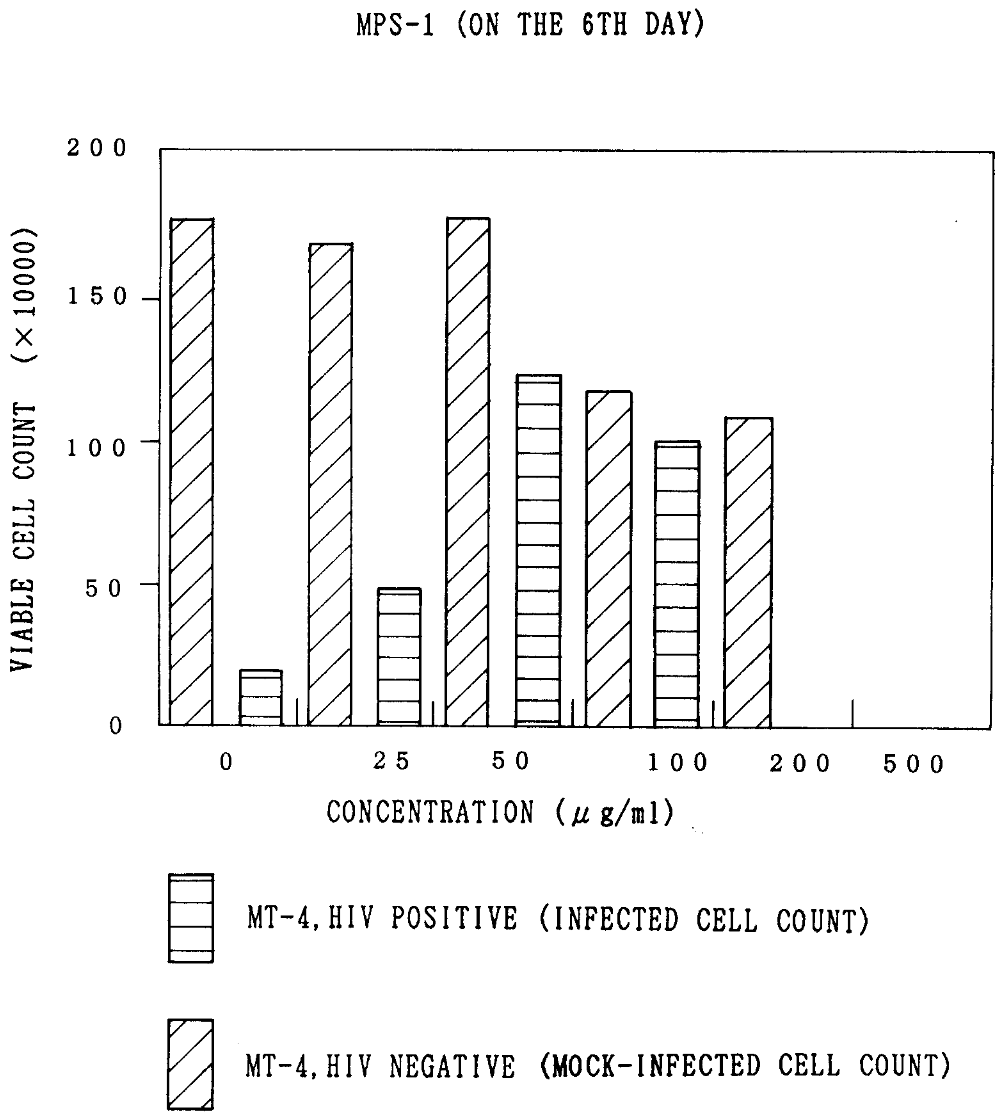
MPS-1 (ON THE 6TH DAY)
VIABLE CELL COUNT (×10000)
200
150
100
50
0
0
25
50
100
200
500
CONCENTRATION (μg/ml)
MT-4, HIV POSITIVE (INFECTED CELL COUNT)
MT-4, HIV NEGATIVE (MOCK-INFECTED CELL COUNT)

# FIG.3

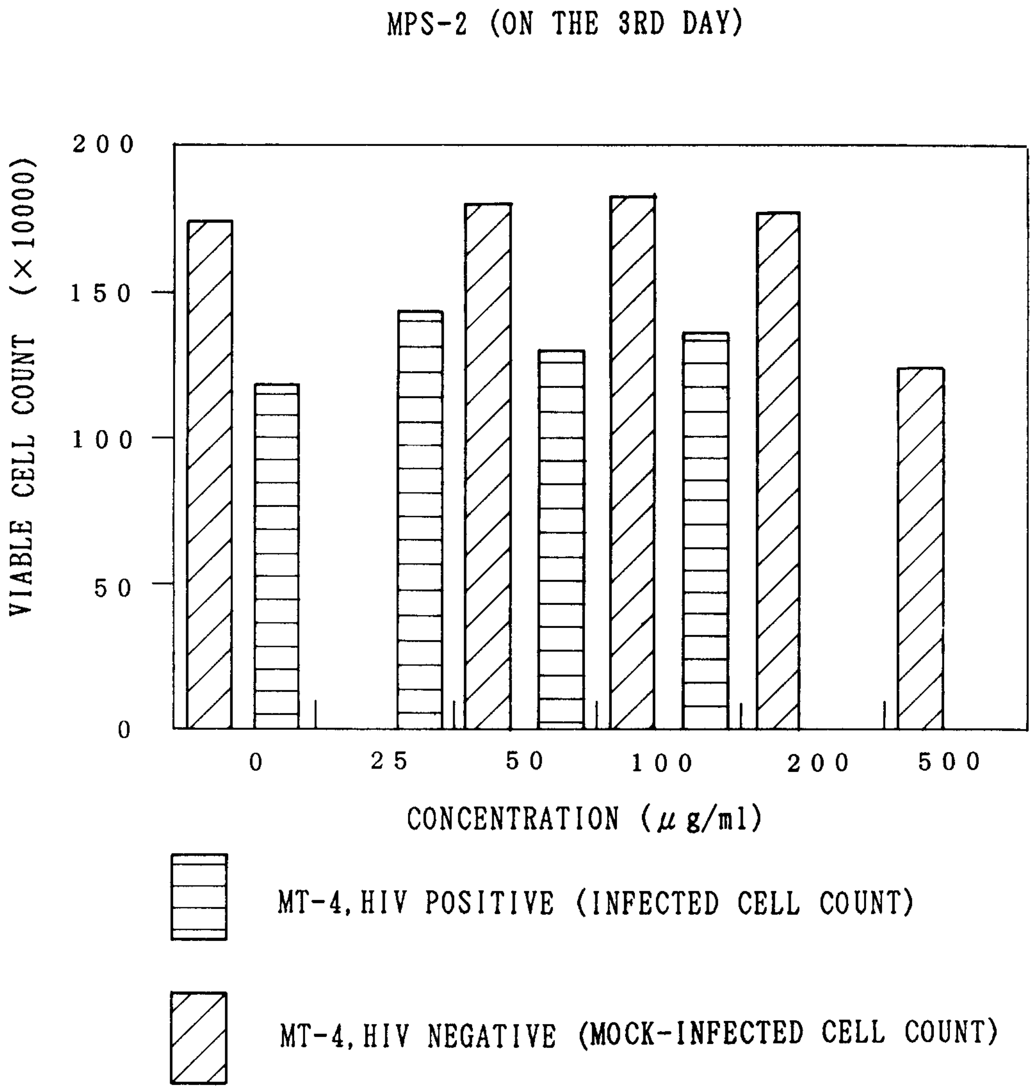
MPS-2 (ON THE 3RD DAY)
VIABLE CELL COUNT (×10000)
200
150
100
50
0
0
25
50
100
200
500
CONCENTRATION (μg/ml)
MT-4, HIV POSITIVE (INFECTED CELL COUNT)
MT-4, HIV NEGATIVE (MOCK-INFECTED CELL COUNT)

# FIG.4

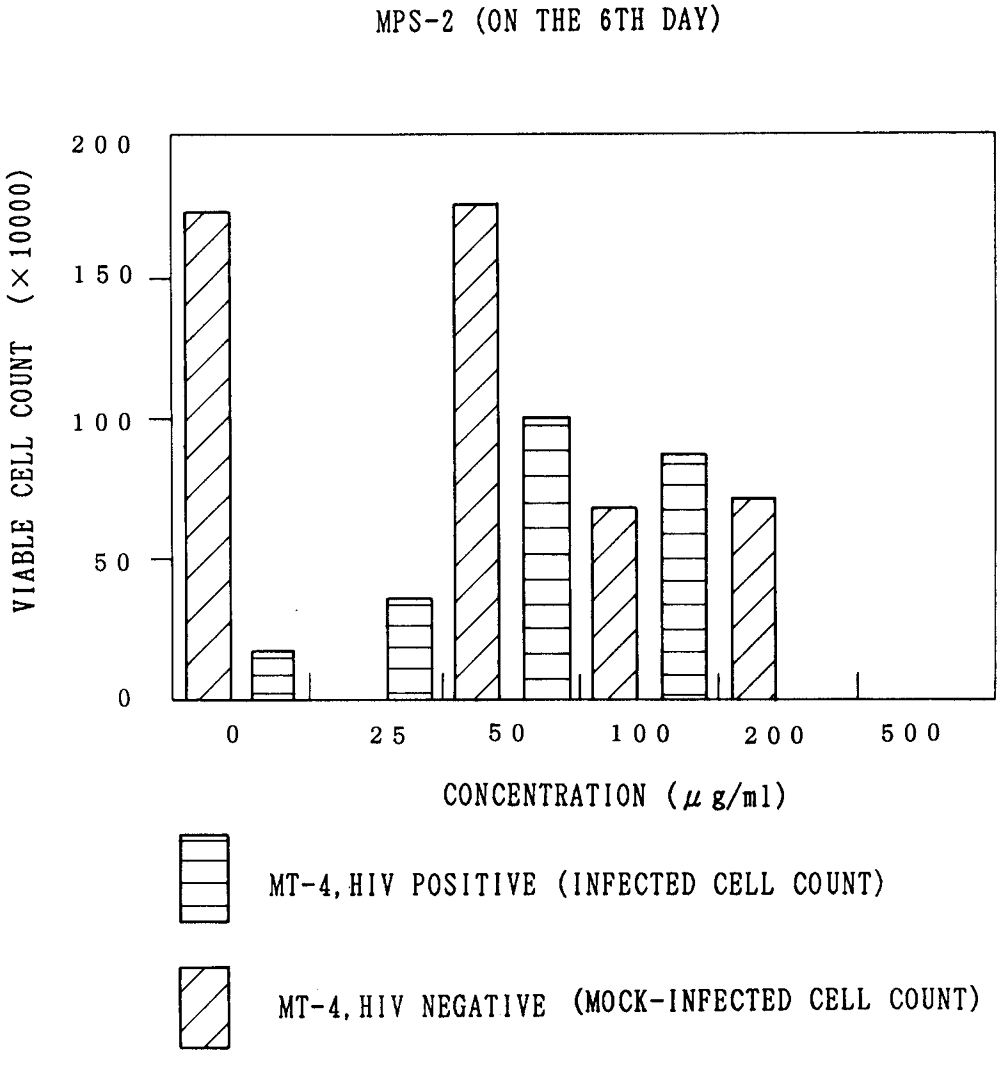
MPS-2 (ON THE 6TH DAY)
VIABLE CELL COUNT (×10000)
200
150
100
50
0
0
25
50
100
200
500
CONCENTRATION (μg/ml)
MT-4, HIV POSITIVE (INFECTED CELL COUNT)
MT-4, HIV NEGATIVE (MOCK-INFECTED CELL COUNT)

# FIG.5

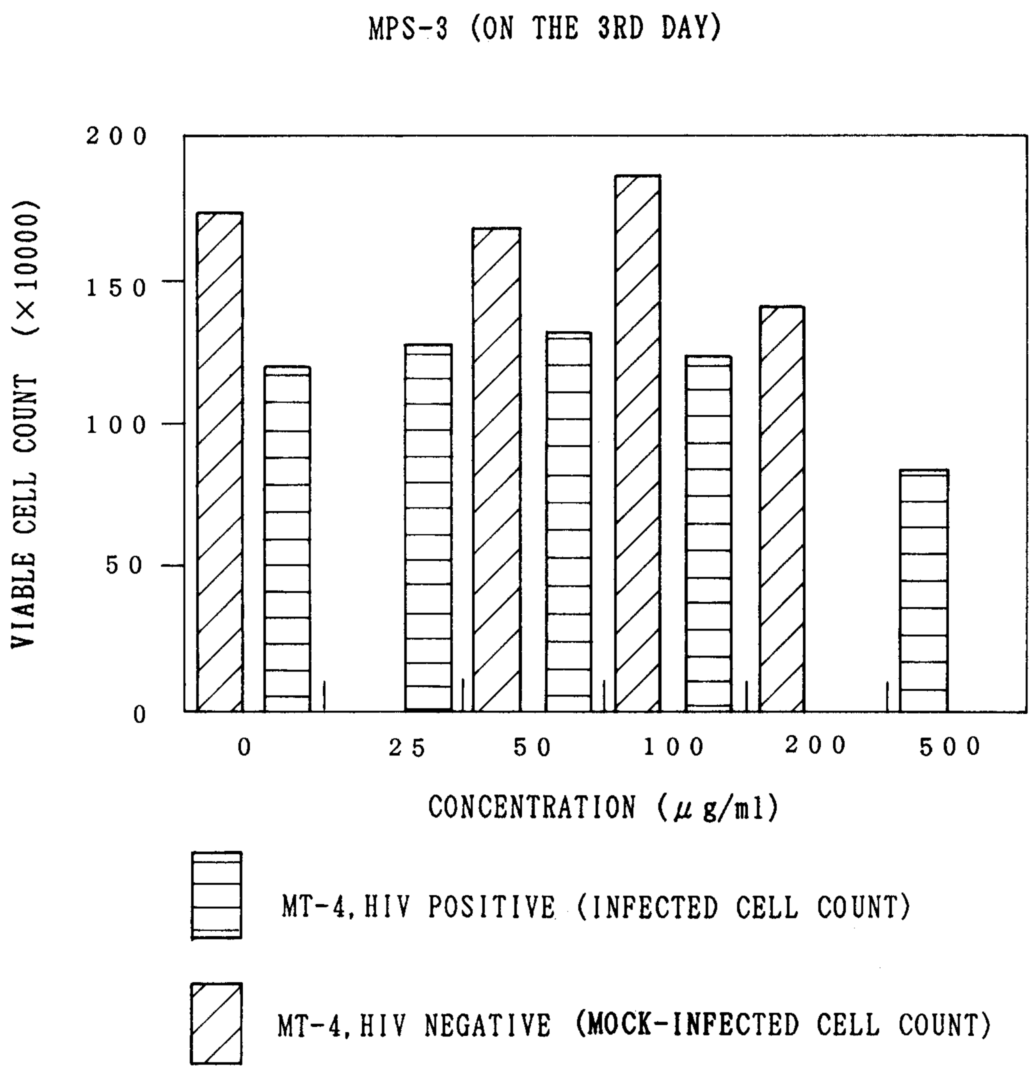
MPS-3 (ON THE 3RD DAY)
VIABLE CELL COUNT (×10000)
200
150
100
50
0
0
25
50
100
200
500
CONCENTRATION (μg/ml)
MT-4, HIV POSITIVE (INFECTED CELL COUNT)
MT-4, HIV NEGATIVE (MOCK-INFECTED CELL COUNT)

# FIG.6

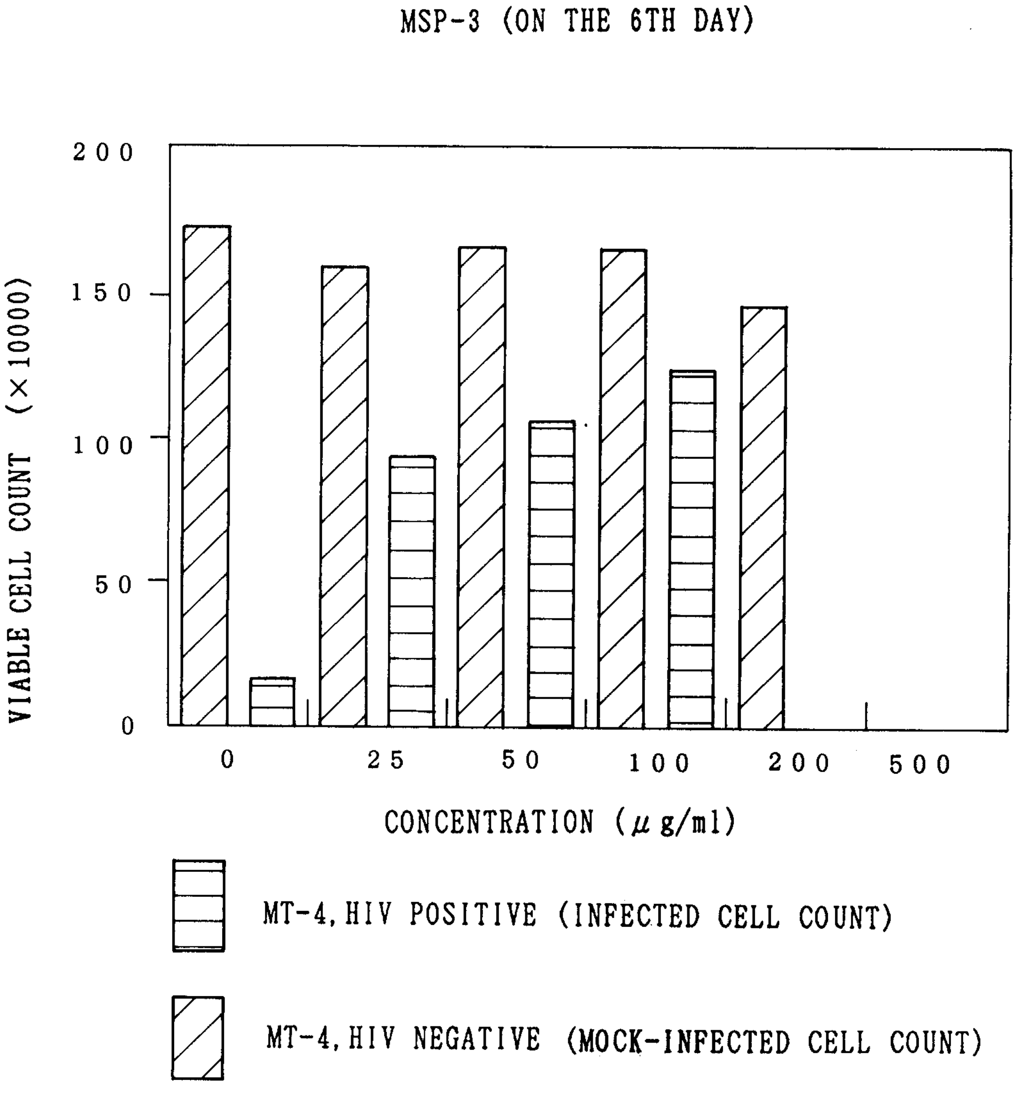
MSP-3 (ON THE 6TH DAY)
VIABLE CELL COUNT (×10000)
200
150
100
50
0
0
25
50
100
200
500
CONCENTRATION (μg/ml)
MT-4, HIV POSITIVE (INFECTED CELL COUNT)
MT-4, HIV NEGATIVE (MOCK-INFECTED CELL COUNT)

FIG.7

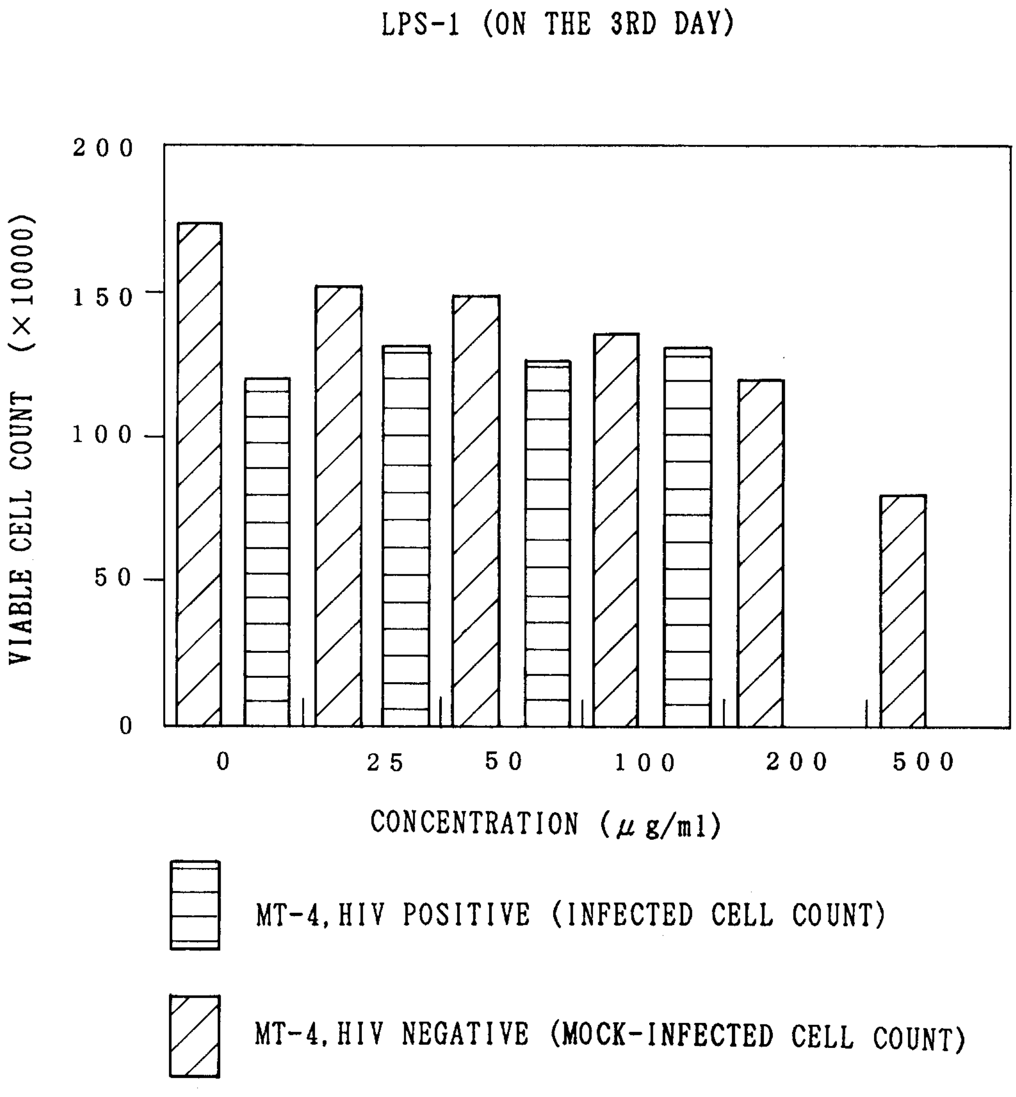
LPS-1 (ON THE 3RD DAY)
VIABLE CELL COUNT (×10000)
200
150
100
50
0
0
25
50
100
200
500
CONCENTRATION (μg/ml)
MT-4, HIV POSITIVE (INFECTED CELL COUNT)
MT-4, HIV NEGATIVE (MOCK-INFECTED CELL COUNT)

# FIG.8

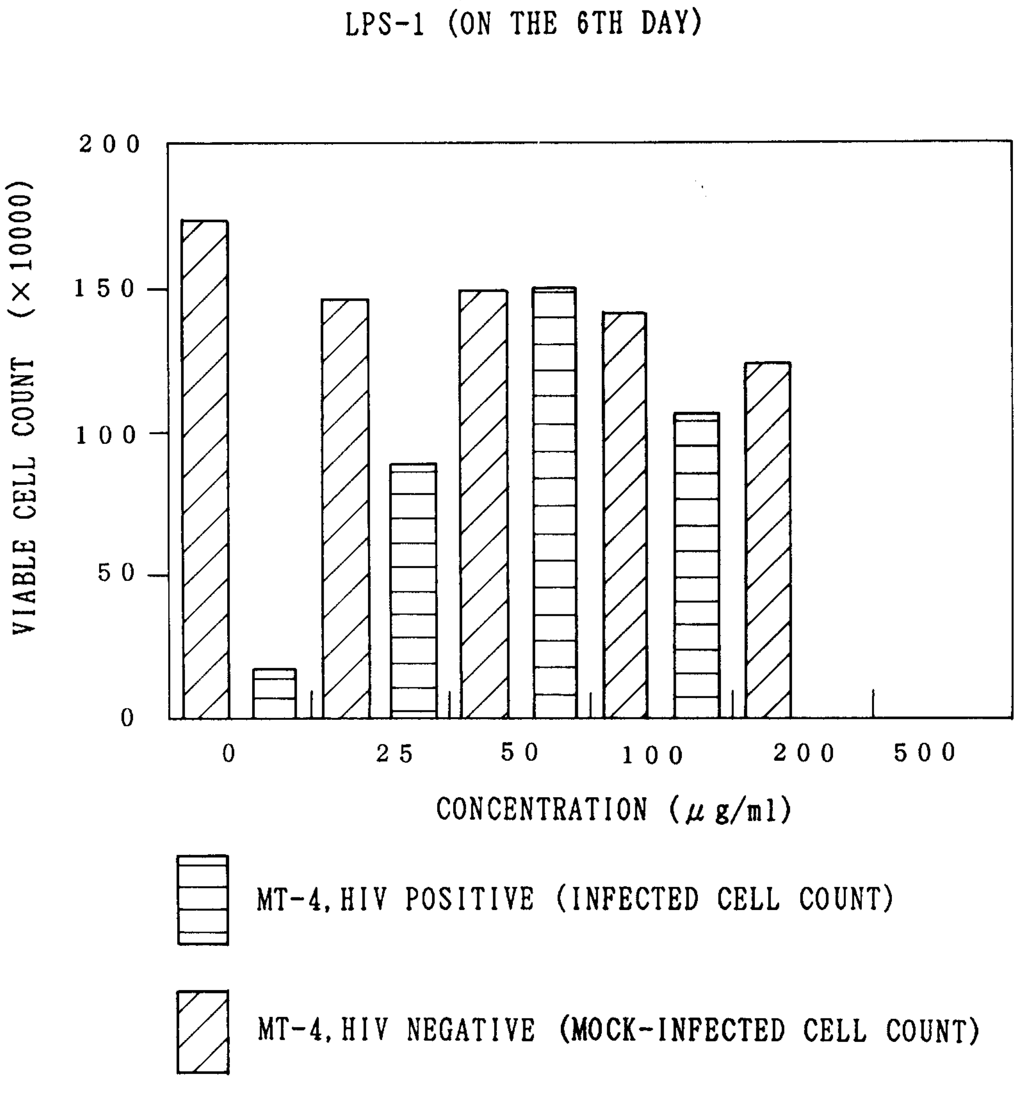
LPS-1 (ON THE 6TH DAY)
VIABLE CELL COUNT (×10000)
200
150
100
50
0
0
25
50
100
200
500
CONCENTRATION (μg/ml)
MT-4, HIV POSITIVE (INFECTED CELL COUNT)
MT-4, HIV NEGATIVE (MOCK-INFECTED CELL COUNT)

# INTERNATIONAL SEARCH REPORT

International Application No PCT/JP91/01122

**I. CLASSIFICATION OF SUBJECT MATTER** (if several classification symbols apply, indicate all) [6]

According to International Patent Classification (IPC) or to both National Classification and IPC

Int. $Cl^5$ C07H15/04, A61K31/70

**II. FIELDS SEARCHED**

Minimum Documentation Searched [7]

| Classification System | Classification Symbols |
|---|---|
| IPC | C07H11/00, C07H15/00-15/18, A61K31/70-31/735 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| P | JP, A, 3-145496 (Dainippon Ink and Chemicals Inc.), June 20, 1991 (20. 06. 91), (Family: none) | 1-11 |
| P | JP, A, 3-145425 (Dainippon Ink and Chemicals Inc.), June 20, 1991 (20. 06. 91), (Family: none) | 12-31 |
| X | JP, A, 2-164890 (Huels AG), June 25, 1990 (25. 06. 90), & EP, A2, 363601 & DE, A1, 3834911 & US, A, 4977253 | 1-11 |
| A | JP, A, 1-100127 (Stiching Rega V.Z.W.), June 2, 1989 (02. 06. 89), & EP, A2, 293826 | 12-31 |
| A | JP, A, 62-215529 (Max Plank), September 22, 1987 (22. 09. 87), Refer to Example 5 & EP, A2, 232744 & DE, A, 3601136 | 12-31 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| October 31, 1991 (31. 10. 91) | November 18, 1991 (18. 11. 91) |
| **International Searching Authority** | **Signature of Authorized Officer** |
| Japanese Patent Office | |